# EUROPEAN PATENT SPECIFICATION

(11) **EP 2 688 470 B1**
(45) Date of publication and mention of the grant of the patent: **04.11.2020**
(21) Application number: 12761286.9
(22) Date of filing: 23.03.2012
(51) Int. Cl.: A61B 18/14, A61B 34/00

(54) **MERGED IMAGE USER INTERFACE AND NAVIGATIONAL TOOL FOR REMOTE CONTROL OF SURGICAL DEVICES**
BENUTZEROBERFLÄCHE FÜR KOMBINATIONSBILDER UND NAVIGATIONSINSTRUMENT ZUR FERNSTEUERUNG CHIRURGISCHER VORRICHTUNGEN
INTERFACE UTILISATEUR À IMAGE FUSIONNÉE ET OUTIL DE NAVIGATION POUR TÉLÉCOMMANDER DES INSTRUMENTS CHIRURGICAUX

(30) Priority: 23.03.2011 US 201113069472; 23.03.2011 US 201113069497
(43) Date of publication of application: 29.01.2014
(73) Proprietor: Acessa Health Inc., Austin, TX 78746 (US)
(72) Inventor: EPSTEIN, Gordon, Livermore CA 94551 (US); SPERO, Richard, Pleasanton CA 94566 (US)
(74) Representative: Lantos, Mihaly
(86) International application number: PCT/US2012/030456
(87) International publication number: WO 2012/129542

(56) References cited:
- WO-A1-2009/065034
- WO-A2-2007/129308
- WO-A2-2010/011781
- US-A- 6 139 546
- US-A1- 2005 097 191
- US-A1- 2005 240 882
- US-A1- 2008 228 166
- US-A1- 2008 228 180
- US-A1- 2008 253 529
- US-A1- 2009 187 230
- US-A1- 2009 198 111
- US-A1- 2009 221 998
- US-A1- 2010 063 509
- US-A1- 2010 228 249
- US-A1- 2010 228 249
- US-A1- 2010 312 094
- US-A1- 2011 015 496

## Description

### FIELD OF THE INVENTION

The invention relates to a system for remote control of a radio frequency ablation surgical device, wherein the ablation device is intended to be positioned in a patient's body for ablation of a tumor, such as a uterine fibroid and, more particularly, to a control button array for navigation through a graphical user interface for remotely controlling a source of radio frequency (RF) energy coupled to an ablation probe during a surgical procedure while maintaining the sterile field.

### BACKGROUND OF THE INVENTION

Advances in technology have resulted in systems that allow a practitioner or other medical professional to remotely control the operation of a medical device. Current devices control an ablation device by direct control of a number of parameters through the use of a matrix of buttons on the face of an RF power generator. These buttons include a pair of buttons labeled with up arrow and down arrow markings to control the setting of temperature which is displayed on a digital meter proximate thereto. A similar pair of buttons is used to control the setting of time. Operation of the buttons to adjust temperature, time and other RF generator functions controlled by the matrix of controls on the front panel of the generator may be performed by a nurse or other person assisting the surgeon. The application of RF energy is controlled by a foot pedal.

US2010/228249 describes user interfaces for electrosurgical tools in robotic surgical systems, in which the surgeon is provided with a high number of control functions through an interface and an intelligent display, however, the use of the system takes place during the surgery and the surgeon has to observe and control the surgical process and he has to follow with his attention and movements the real time image how the manipulated tools carry out the job and there are very few or no time before the surgery to adjust the parameters under which the surgery has to be carried out.

In ablation tasks prior to the actual execution of the job the area where ablation should be carried out is discovered and mapped in detail, and a number of information is available for the user that can provide assistance for different adjustments how the ablation should be carried out.

There are sophisticated ablation devices that can be used for executing the intervention. A typical example is disclosed in the publication WO 2009/065034 wherein in and around a trocar a plurality of stylets are used which include thermocouples to sense the local temperature where their ends contact human tissues, and the information sensed can be used in adjusting the ablation process.

A further publication US2008/228180 describes an ablation device wherein four leg electrode pads are used as return electrodes to provide heat prevention.

### Object of the Invention

There is a need for an improved remote control system for radio frequency ablation devices, which can utilize the fact that prior to the execution of the ablation process and in the knowledge of several properties related to the required ablation the user can adjust circumstances and parameters of the ablation, whereby during the intervention his attention can be focused on steps that should be carried out on the spot, and a virtual image can assist him in orientation concerning the adjusted or measured parameters.

### Summary of the Invention

This task has been resolved by the present invention by providing a system for remote control of a radio frequency ablation surgical device structured as defined in the attached claims.

### BRIEF DESCRIPTION OF THE DRAWINGS

A full understanding of the invention can be gained from the following description of the preferred embodiments when read in conjunction with the accompanying drawings in which:
Fig. 1a illustrates an ablation device useful with the inventive system;
Fig. 1b illustrates an ablation system incorporating computer controls in accordance with the inventive system;
Fig. 1c shows the graphical user interface screen in which menus "fibroid data", "descriptor", "summary", "select procedure", "ready ablate" and "ready coag" are displayed, and the navigational tool has scrolled to the menu choice "fibroid data";
Fig. 2 shows the graphical user interface screen in which the menu "fibroid data" is selected to show submenus "number", "diameter", "position" and "type", and in which "1" is selected as the number;
Fig. 3 shows the graphical user interface screen in which the menu "fibroid data" and the submenu "number" are selected, and in which "2" is selected as the number;
Fig. 4 shows the graphical user interface screen in which the menu "fibroid data" is selected, the submenu "diameter" is selected and a further submenu "< 1 cm" is selected;
Fig. 5 shows the graphical user interface screen in which the menu "fibroid data" is selected, the submenu "diameter" is selected and further submenu choices are shown, which range from "< 1 cm" to "15";
Fig. 6 shows the graphical user interface screen in which the menu "fibroid data" is selected, the submenu "position" is selected and further submenu choices "anterior", "posterior" and "lateral" are shown;
Fig. 7 shows the graphical user interface screen in which the menu "fibroid data" is selected, the submenu "position" is selected, and the further submenu choices "midline", "right" and "left" are shown;
Fig. 8 shows the graphical user interface screen in which the menu "fibroid data" is selected, the submenu "position" is selected and the further submenu choices "fundal", "miduterine" and "lower segment" and "cervical" are shown;
Fig. 9 shows the graphical user interface screen in which the menu "fibroid data" is selected, the submenu "diameter" is selected with the submenu " < 1 cm" selected, the submenu "position" selected with the submenu "anterior" selected and the further submenu "type" showing the choices "intramural", "subserosal", "submucous 1" and "submucous 2";
Fig. 10 shows the graphical user interface screen in which the navigational tool has scrolled to the menu choice "descriptors";
Fig. 11 shows a table containing exemplary patient fibroid tumor data displayed on the graphical user interface when the menu "descriptors" is selected;
Fig. 12 shows the graphical user interface screen in which the navigational tool has scrolled to the menu choice "summary";
Fig. 13 shows a graphical compilation of exemplary patient fibroid tumor ablation data displayed on the graphical user interface when the menu "summary" is selected;
Fig. 14 shows the graphical user interface screen in which the navigational tool has scrolled to the menu choice "select procedure";
Fig. 15 shows the graphical user interface screen in which the menu "select procedure" is selected, the submenu "control mode" is selected, and the submenus "temperature" and "manual" are shown;
Fig. 16 shows the graphical user interface screen in which the menu "select procedure" is selected, the submenu "control mode" is selected, and the submenu "temperature" is shown with its submenus 90°, 95° and 100°;
Fig. 17 shows the graphical user interface screen in which the menu "select procedure" is selected, the submenu ""control mode" is selected, a further submenu "temperature" is selected, and a temperature target of 100° is selected;
Fig. 18 shows the graphical user interface screen in which the menu "select procedure" is selected, the submenu ""manual" is selected, and the submenu target power levels of "15" and "10" are displayed;
Fig. 19 shows the graphical user interface screen in which the menu "select procedure", the submenu "temperature" and 100° have been selected; and the navigational tool has scrolled to the menu choice "ready ablate";
Fig. 20 shows the graphical user interface screen in which the menu "select procedure", the submenu "temperature" and 100° have been selected; and the menu "ready ablate" has been selected showing the submenu choices "confirm" and "reset;"
Fig. 21 shows the graphical user interface screen in which the menu "select procedure", the submenu "manual" and power level target "15" have been selected; and the navigational tool has scrolled to the menu choice "ready ablate";
Fig. 22 shows the graphical user interface screen in which the menu "select procedure", the submenu "manual" and power level target "15" have been selected; and the menu "ready ablate" has been selected showing the submenu choices "confirm" and "reset;"
Fig. 23 shows the graphical user interface screen in which the menu "select procedure", the submenu "temperature" and the temperature of "100°" have been selected at the start of ablation of the fibroid tumor; also shown is the average temperature and the temperature in tissue adjacent to each of the stylets of the ablation device as well as the temperature in right and left pads placed on a patient's legs;
Fig. 24 shows the graphical user interface screen in which the menu "select procedure", the submenu "manual" and the target power level of "15" have been selected at the start of ablation of the fibroid tumor; also shown is the average temperature and the temperature in tissue adjacent to each of the stylets of the ablation device, the power level submenu as well as the temperature in the right and left pads placed on a patient's legs;
Fig. 25 shows the graphical user interface screen in which the menu "select procedure", the submenu "temperature" and the temperature of "100°" have been selected after 18 seconds into the ablation procedure, as shown as "ramp time" and "total time"; also shown is the average temperature and the temperature in tissue adjacent to each of the stylets of the ablation device, the power level submenu as well as the temperature in the right and left pad placed on a patient's legs;
Fig. 26 shows the graphical user interface screen in which the menu "select procedure", the submenu "temperature" and the temperature of "100°" has been selected after 31 seconds into the ablation and 13 seconds at the target temperature for a total time of 43 seconds, as shown as "ramp time", "target time" and "total time"; also shown is the average temperature and the temperature in tissue adjacent to each of the stylets of the ablation device, the power level submenu as well as the temperature in the right and left pads placed on a patient's legs;
Fig. 27 shows the graphical user interface screen in which the menu "select procedure", the submenu "manual" and the target power level submenu of "15" has been selected after 1 second into the ablation. as shown as the target time; also shown is the average temperature and the temperature in tissue adjacent to each of the stylets of the ablation device as well as the temperature in the right and left pads;
Fig. 28 shows the graphical user interface screen in which the menu "select procedure", the submenu "temperature" and 100° has been selected at the start of coagulation of the trocar entry path; also shown is the target power level submenu set at 8 watts;
Fig. 29 shows the graphical user interface screen in which the menu "select procedure", the submenu "temperature" and 100° has been selected after 1 second of coagulation; also shown is the target power level submenu set at 8 watts;
Fig. 30 shows the graphical user interface screen in which the menu "select procedure", the submenu "manual" and the target power level "15" has been selected at the start of coagulation;
Fig. 31 shows the graphical user interface screen in which the menu "select procedure", the submenu "manual" and target power level of "15" has been selected after 1 second of coagulation;
Fig. 32 shows the graphical user interface screen in which the menus "return", "sound", and "patient" are displayed and "return" has been selected showing patient and computer software information;
Fig. 33 shows the graphical user interface screen in which the menus "return", "sound", and "patient" are displayed and the navigational tool has scrolled to the "sound" menu;
Fig. 34 shows the graphical user interface screen in which submenus"1" through "15" are displayed after selecting the "sound" menu and showing the sound volume selected as "12";
Fig. 35 shows the graphical user interface screen in which the menus "return", "sound", and "patient" are displayed and the navigational tool has scrolled to the "patient" menu;
Fig. 36 shows the graphical user interface screen in which the submenu identification number of a patient is displayed after selecting the "patient" menu;
Fig. 37 shows the graphical user interface screen in which the menus "return" and "shutdown" are displayed;
Fig. 38 shows the graphical user interface screen in which the menus "return" and "shutdown" are displayed and "shutdown" is selected;
Fig. 39 shows the graphical user interface screen in which the menus "return" and "shutdown" are displayed, "shutdown" is selected and the submenus "yes" and "no" are displayed;
Fig. 40 shows the graphical user interface screen in which the computer software program has been shut down after selecting "yes" from the "shutdown" menu;
Figure 41 illustrates an alternative inventive system where imaging data is displayed on the GUI; and
Figure 42 illustrates an alternative inventive system where imaging data from two different image sources is merged and displayed on the GUI.

### DETAILED DESCRIPTION OF THE INVENTION

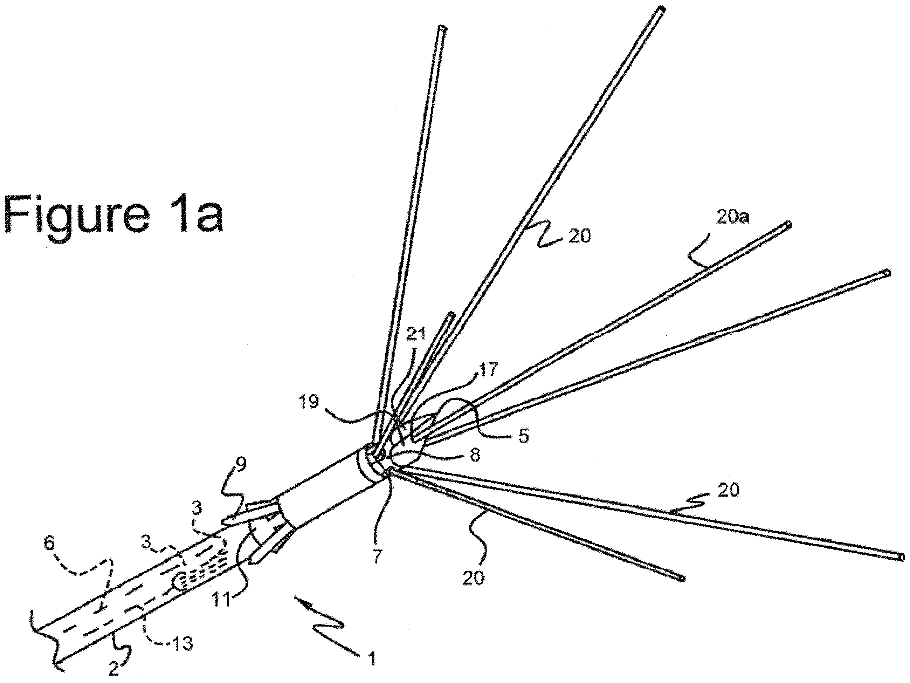

Fig. 1a is a perspective view of a multiple antennae or stylet ablation trocar instrument 1 useful in practicing the inventive system. Ablation instrument 1 comprises a cannula 2 which houses a plurality of stylets 20 and, optionally, a plurality of anchors 4. A trocar point 5 is provided at the distal end of cannula 2. At least one conductor 6 is provided within cannula 2. Conductor 6 is electrically coupled to stylets 20 and trocar point 4 and accordingly provides RF energy to stylets 20 and trocar point 5. In accordance with the invention, stylets 20 and trocar point 5 are electrically coupled to each other and electrically isolated from other exposed portions of ablation instrument 1. Stylets 20 and trocar point 5 are at the distal end of ablation instrument 1. Each of the stylets is made of thin wire-like tubular members and during the procedure is initially housed entirely within the cannula 2.

Stylets 20 are deployed for ablation by being advanced in the forward direction toward the distal end of ablation instrument 1 out from ablation instrument 1 through openings 7. As stylets 20 are advanced through openings 7, they bear against deflection surfaces 8. Deflection surfaces 8 are defined in the metal body which defines trocar point 5 at the distal end of the cannula 2.

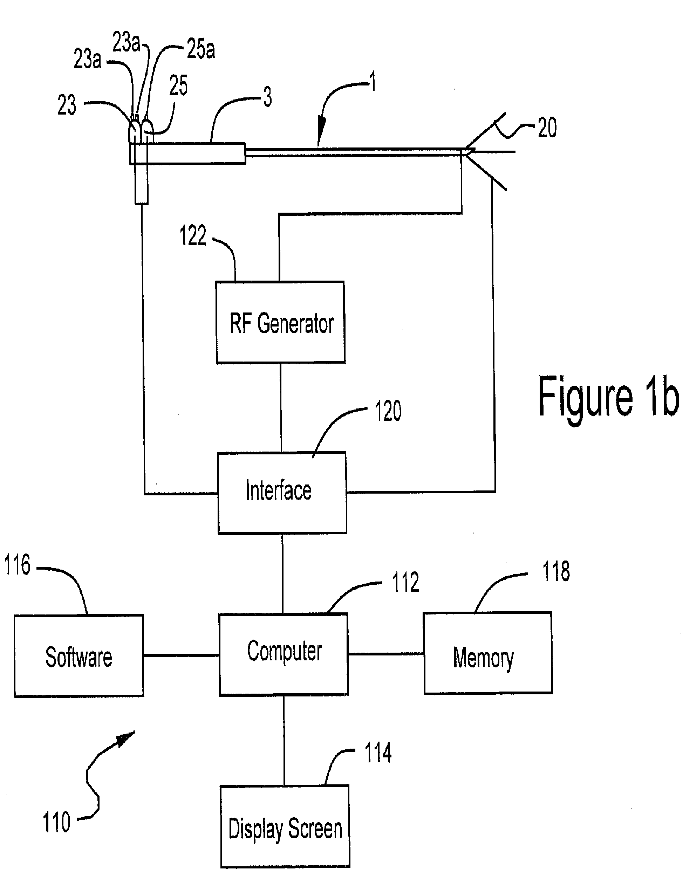

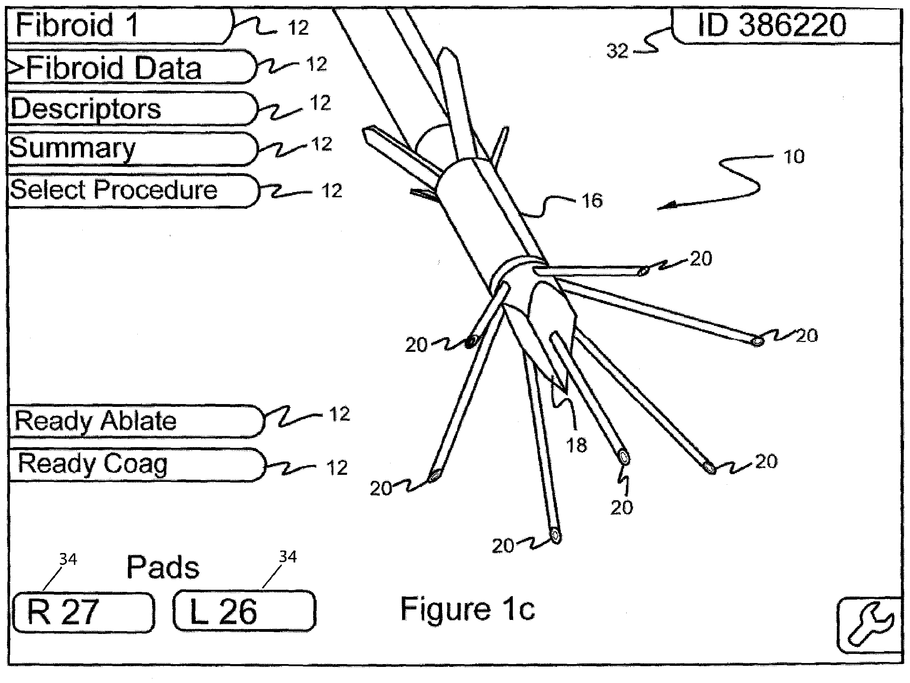

During use of the inventive system, trocar point 5 at the distal end of cannula 2 is used to initially pierce the tissue of the fibroid tumor during use of the inventive ablation device 1. Optionally, a plurality of anchors 9, also housed within ablation instrument 1, may be deployed rearwardly toward the proximal end of ablation instrument 1. During deployment, anchors 9 are deflected by deflection surface 11 to move into the positions illustrated in Figure 1. After deployment, anchors 9 may optionally be used to prevent rearward movement of trocar point 5 during deployment of stylets 20.

Stylets 20 are deployed through the use of a slideably mounted operator member 13 housed within cannula 2 and coupled to an operating handle at its proximal end. Anchors 9 are also deployed through the use of a slideably mounted operator member (not illustrated) housed within cannula 2 and coupled to an operating handle at its proximal end. The distal end of operator member 13 is coupled to stylets 3 which may thus be advanced an identical distance in unison. The retraction and deployment of anchors and stylets is controlled by an operator handle 3 as illustrated in Fig. 1b.

Referring to Fig. 1c, a graphical user interface (GUI) 10 display screen in accordance with the present invention is shown. In accordance with the preferred embodiment, a surgeon uses a medical device such as an ablation device. The ablation device is illustrated in GUI 10 by ablation device illustration 16. The ablation device is used for ablating tissue masses. Use of the same is facilitated by GUI 10 and the navigational button matrix to minimize the likelihood of breaking the sterility of the surgical field. The GUI 10 displays a choice of menu items 12 that the practitioner can scroll through by depressing the scroll button 23 (Figure 1b) which carries two raised dots 23a on its surface on the navigational button matrix. All of the menu items 12 are displayed at the same time. The menu items 12 allow the surgeon or other practitioner to enter patient data, collect patient data and perform a surgical procedure all within the sterile field. When a desired menu is reached, the surgeon selects from menu items 12 by depressing the select button 25, which has one raised dot 25a on its top surface, on the navigational button matrix, which may be viewed as a whole as a navigational tool. In one preferred embodiment of the invention, when ablating a tissue mass such as a fibroid tumor, the menu 12 choices comprise the "Fibroid" number data, "Fibroid Data", "Descriptors", "Summary", "Select Procedure", "Ready Ablate" and "Ready Coag". In Figure 1b, the system indicates that information with respect to a first fibroid, "Fibroid 1", is being collected. In Figure 1, an arrow indicator 15 indicates that the surgeon has scrolled to the "Fibroid Data" menu item. Repeated depression of the scroll button causes the arrow indicator to move in sequence through the choices comprising menu items labeled "Fibroid" for the fibroid number, "Fibroid Data", "Descriptors", "Summary", "Select Procedure", "Ready Ablate" and "Ready Coag". Stopping on the fibroid number data which is labeled "Fibroid 1" in Figure 1b (which results in placing the arrow indicator before the indication "Fibroid 1"), and depressing of the select button results in causing the arrow indicator to cursor through indicators reading "Fibroid 1", "Fibroid 2", "Fibroid 3", "Fibroid 4", "Fibroid 5" and so forth. If one next depresses the scroll button, arrow indicator 15 indicates selection of "Fibroid Data". As an alternative, one also can scroll to the "Fibroid Data", push select, scroll to the numbers until the desired fibroid number is presented (for example "Fibroid 2"), and click the select button resulting in the display of "Fibroid 2" instead of "Fibroid 1" as illustrated in Figure 1.

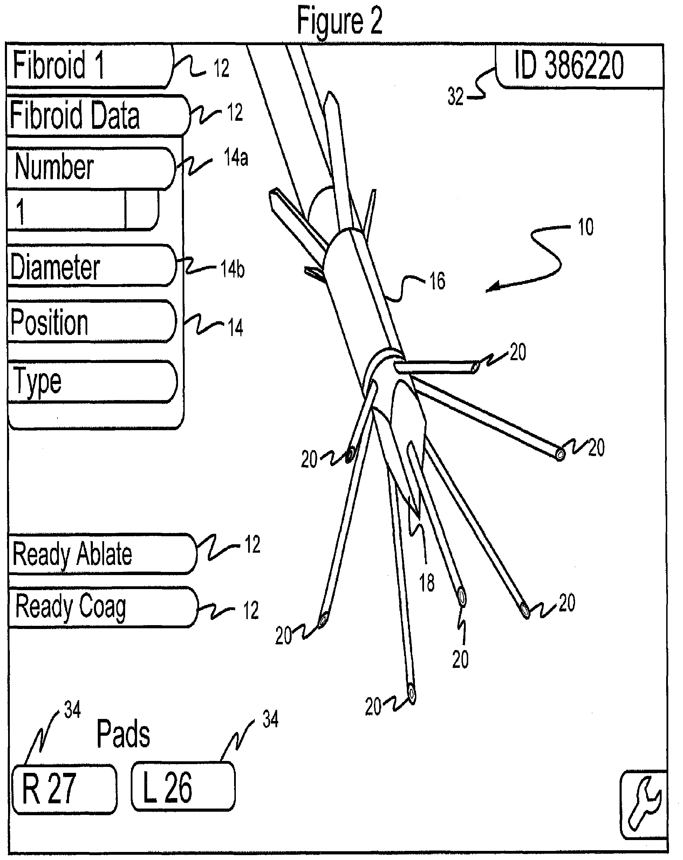

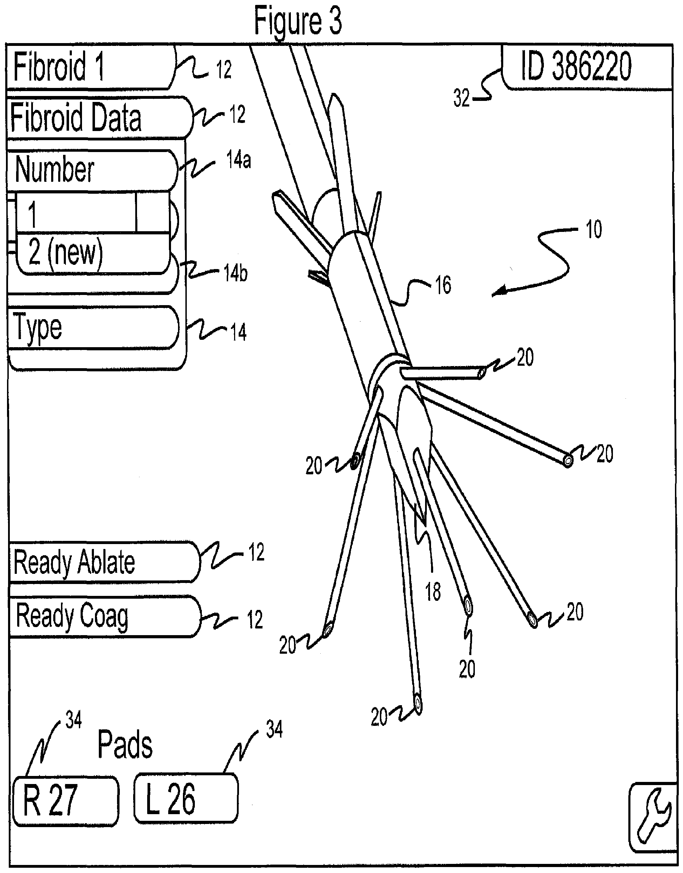

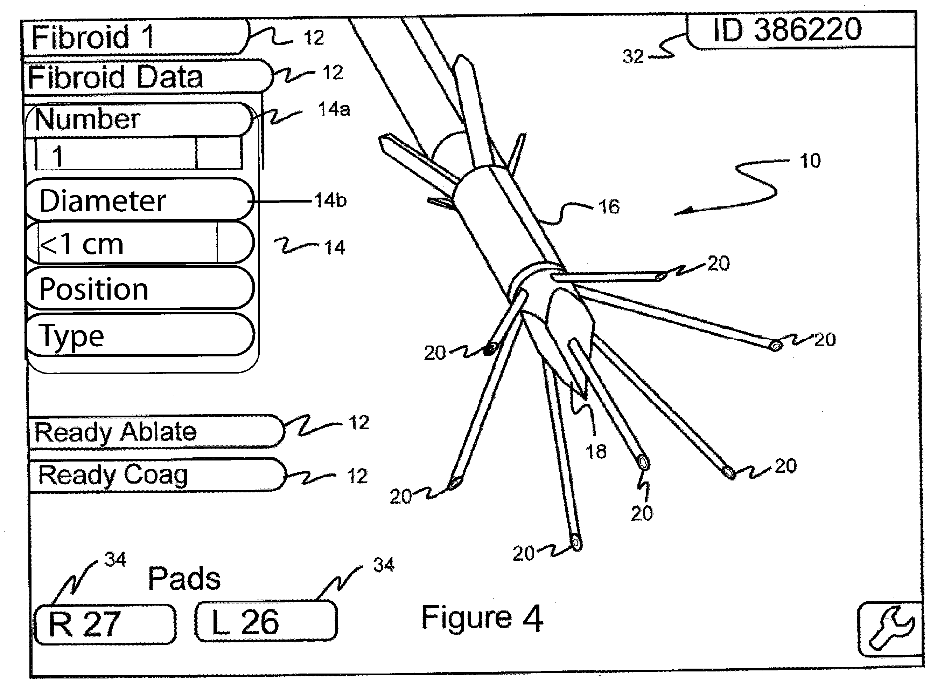

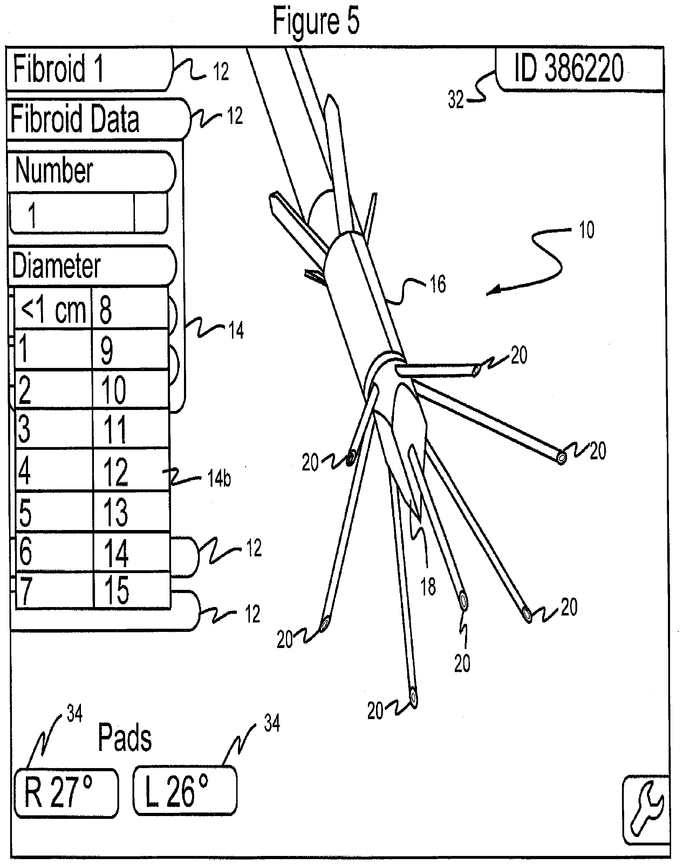

Referring to Figs. 2-9, by scrolling to and selecting "Fibroid Data", a series of choices are presented in the form of a submenu 14. These choices allow for entering data regarding, in the illustrated example, a first uterine fibroid, namely "diameter", "position" and "type". Such information may be gathered by the surgeon based on, for example, ultrasound imaging and laparoscopic imaging. When submenu item 14a, "number", is scrolled to and selected, the practitioner can select the number associated with the fibroid, for example, "1" or "2" (Figs. 2 and 3). When submenu item 14b, "diameter" is selected, as illustrated in Figure 4, the size of the first fibroid is illustrated. Next, if the scroll button is depressed the system moves to the diameter indication "<1 cm". The diameter indication may be highlighted, indicating selection of the diameter indication. It is noted that indications of selection may be arrows, highlighting, or any suitable means. Depressing the select button when the diameter indication is highlighted results in presenting a submenu comprising a series of possible diameter values, as illustrated in Figure 5. More particularly, this menu 14B displays choices ranging from < 1 cm to 15 cm (Fig. 5) from which the practitioner can select, by scrolling to the proper size by repeatedly pressing the scrolling button and then pressing the select button when the proper size is highlighted. In this example, size "<1 cm" has been selected. Alternatively, or in addition, scrolling may be facilitated by grouping each function whereby pushing the scroll button down and keeping it down results in the machine automatically advancing through the sequence of choices.

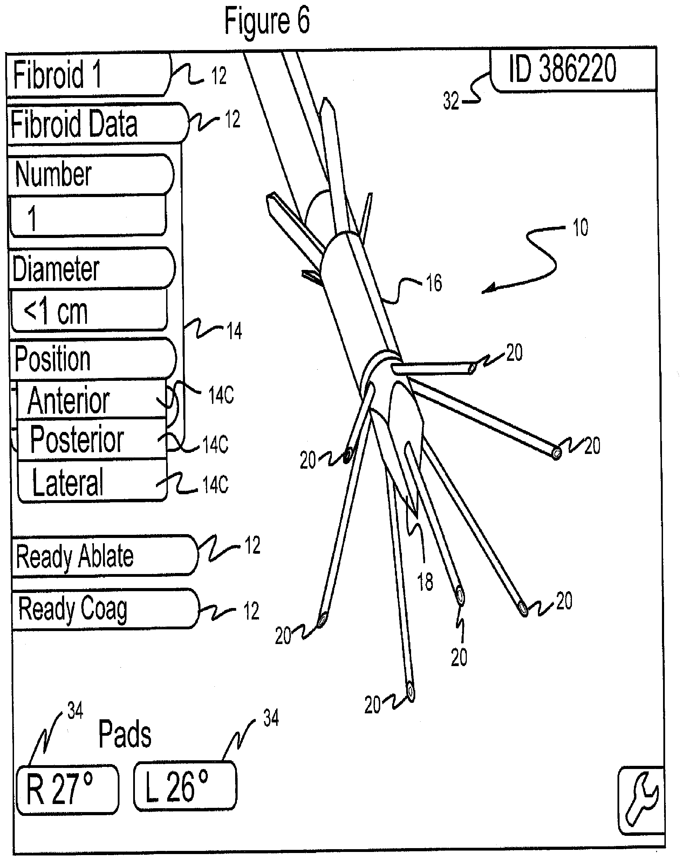

When submenu item 14c "Position" is scrolled to and selected, further submenu 14C, which contains displays of "Anterior", "Posterior" and "Lateral" is displayed, presenting three choices from which the practitioner can select (Fig. 6) in order to make a record of the type of uterine fibroid. In this example, "Anterior" is selected.

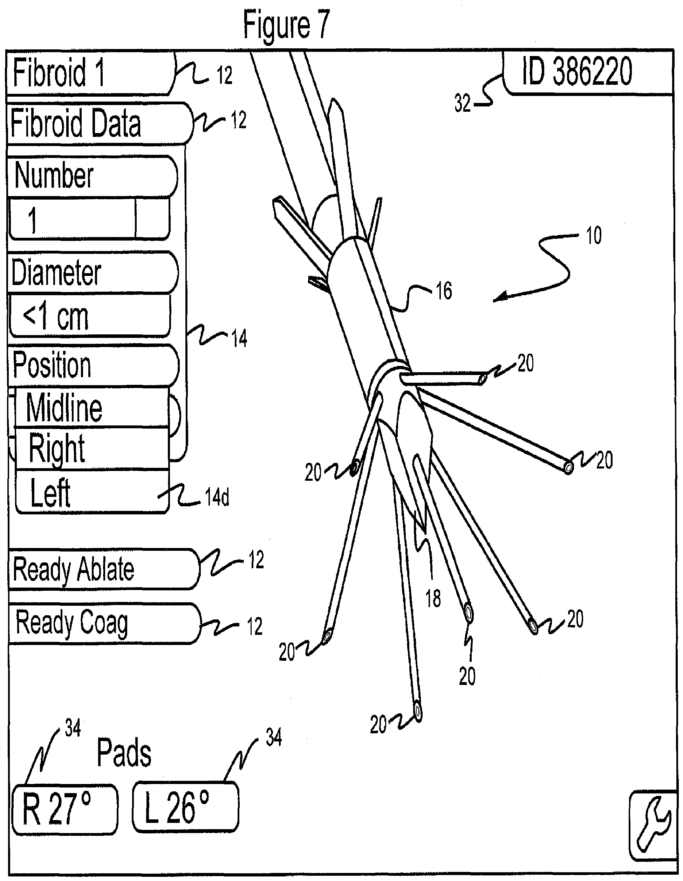

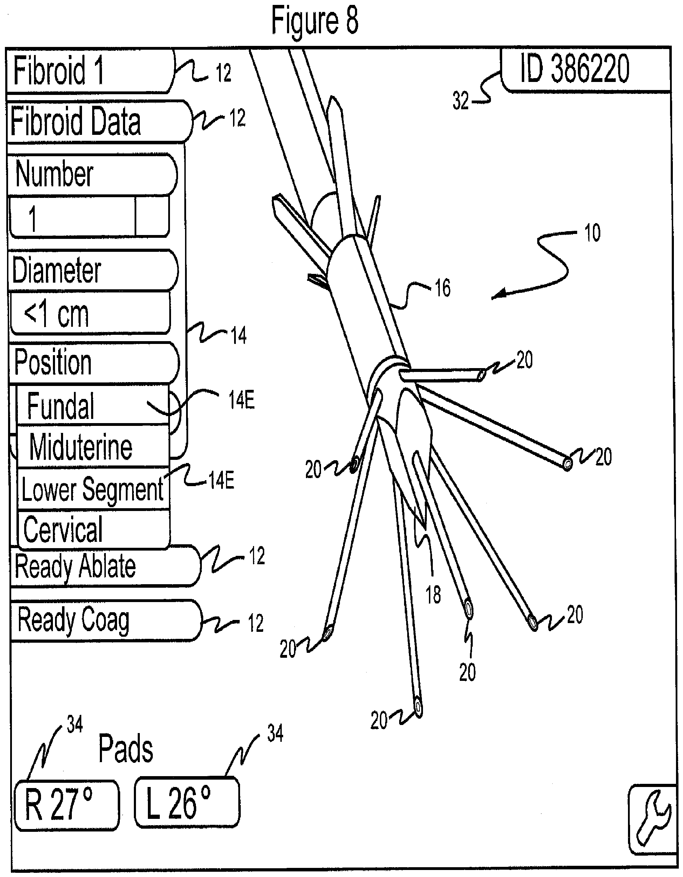

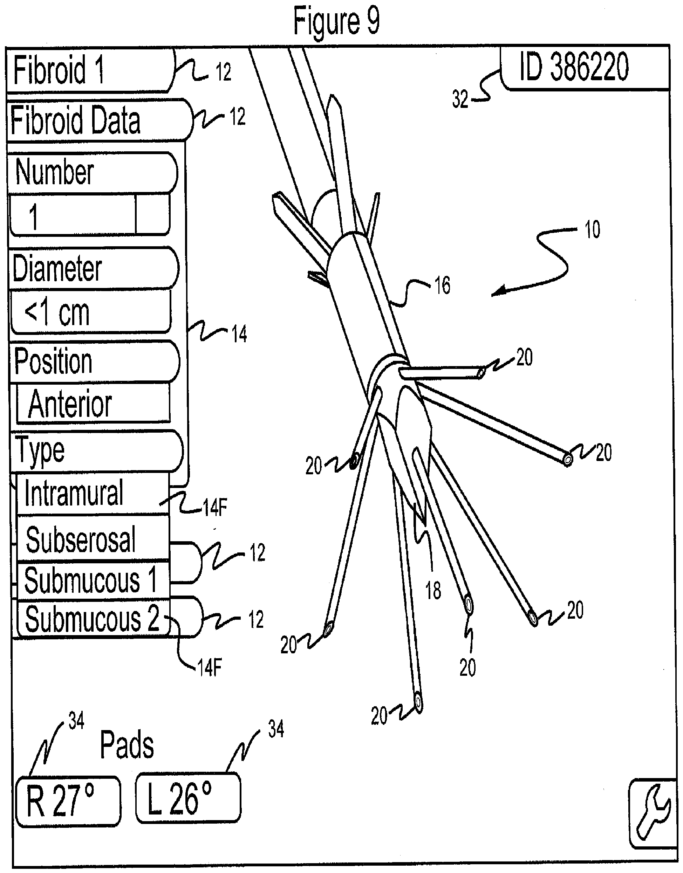

Upon making a selection from one of the items of submenu items 14B, a still further submenu of items 14D is produced, giving menu choices "Midline", "Right" and "Left", to which the practitioner can scroll and select (Fig. 7). In this example, "Midline" is selected. Upon making a selection from one of these submenu items 14D, still further submenu items 14E indicating "Fundal", "Miduterine", "Lower Segment" and "Cervical" are displayed, to which the practitioner can scroll and select (Fig. 8). In this example, "Fundal" is selected. Upon making a selection from one of these submenu items 14E, a still further submenu of items 14F "Intramural", "Subserosal", "Submucous 1" and "Submucous 2" are displayed, to which the practitioner can scroll and select (Fig. 9). In this example, "Intramural" is selected.

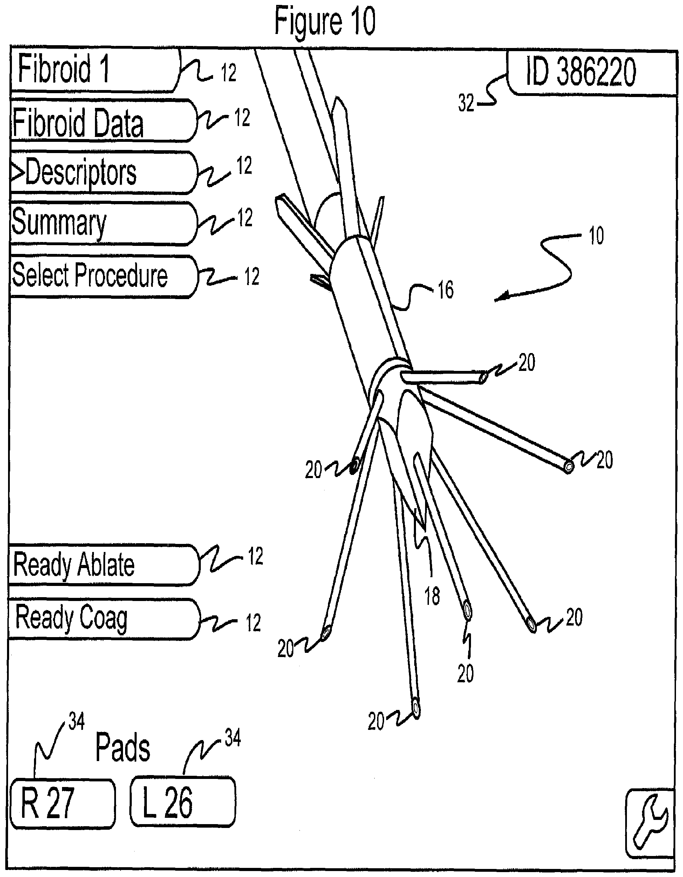

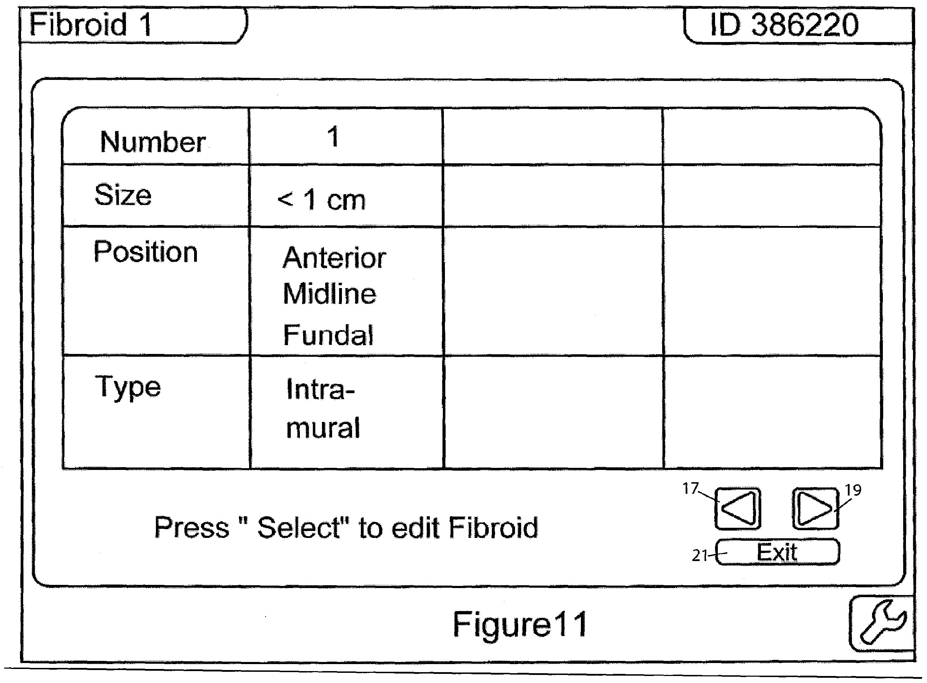

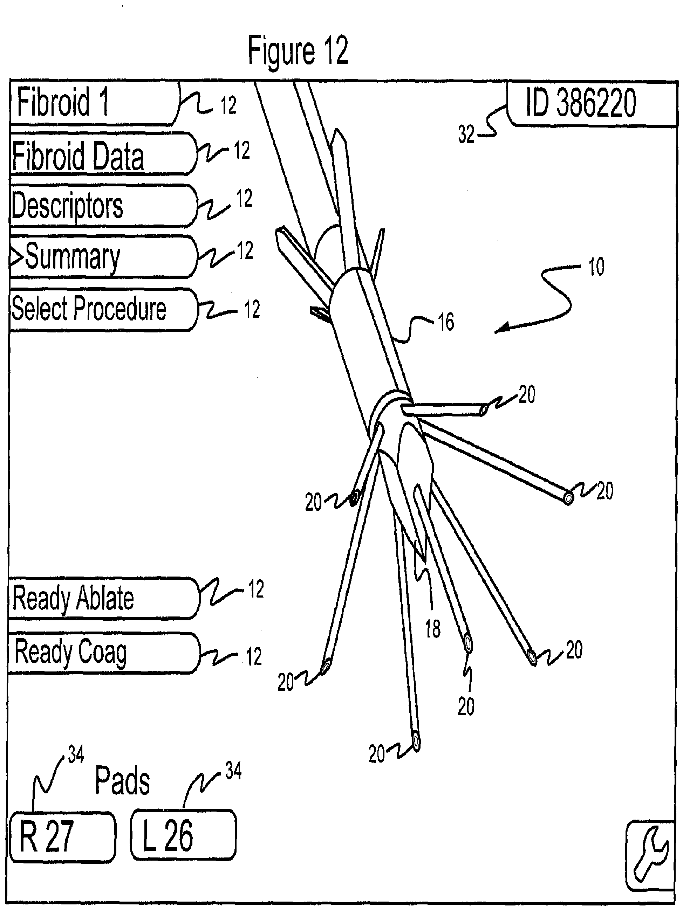

Referring to Figs. 10-13, scrolling to menu 12 "Descriptors" (Fig. 10), and then pushing the select button opens a table (Fig. 11), in which all data selections detailed above that have been inputted in the "Fibroid Data" menu 14 are displayed on the GUI, and which allows input of the data for all fibroids. In this screen, the surgeon may scroll through the table items "Number", "Size", "Position", "Type", and "Exit". Like the other menu items in the inventive device, continuing to depress the scroll button will continue cycling through the menu selections. Accordingly, continuing to cycle through the menu items by pressing the scroll button will result in display of the table items "Number", "Size", "Position", "Type", "Exit", "Number", "Size", "Position", "Type", "Exit", "Number", "Size", "Position", "Type", "Exit", and so forth. Depressing the select button on any of these items will activate the arrow icon 17 and 19, and exit icon 21, which allows editing of the selected menu item. Icons 17 and 19, when highlighted and selected by depression of the select button, actuate navigation through the various choices in opposite order. Icon 21, when highlighted by pressing of the scroll button and selected by depression of the scroll button, causes the system to exit the screen of Figure 11 and go to the screen of Figure 12.

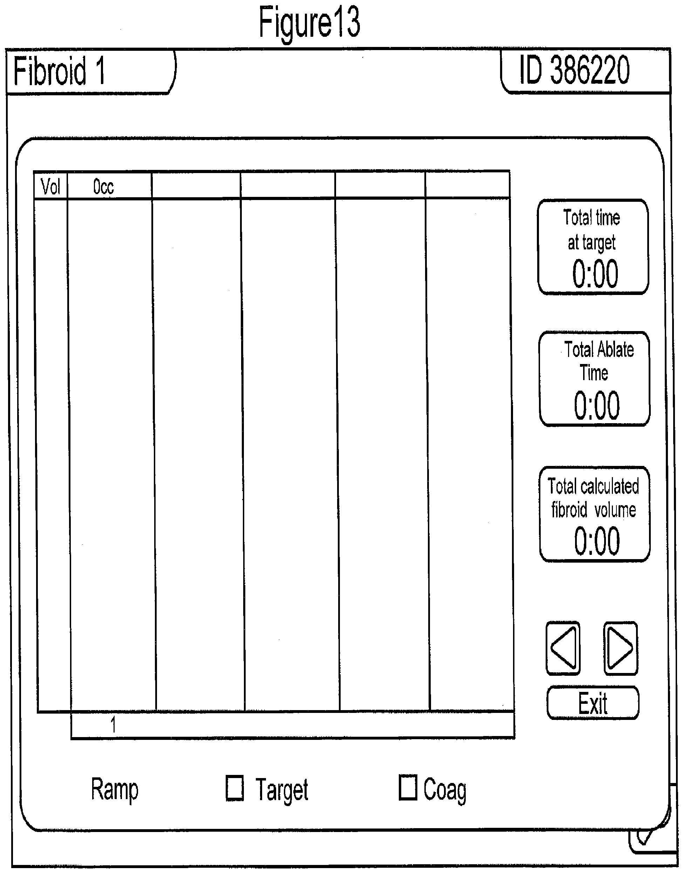

Selecting the menu 12 "Summary" (Fig. 12) opens up a graphical display (Figure 13), in which all data related to a particular fibroid tumor being ablated and associated patient identifier are displayed. This screen does not allow editing of the displayed information.

In general, in accordance with the preferred embodiment, items are scrolled to and selected by depression of the select button. However, in principal, resting the cursor for a particular length of time (for example, one second) can be set in the software to be the equivalent of a select, and this can be reversed by pushing the select button.

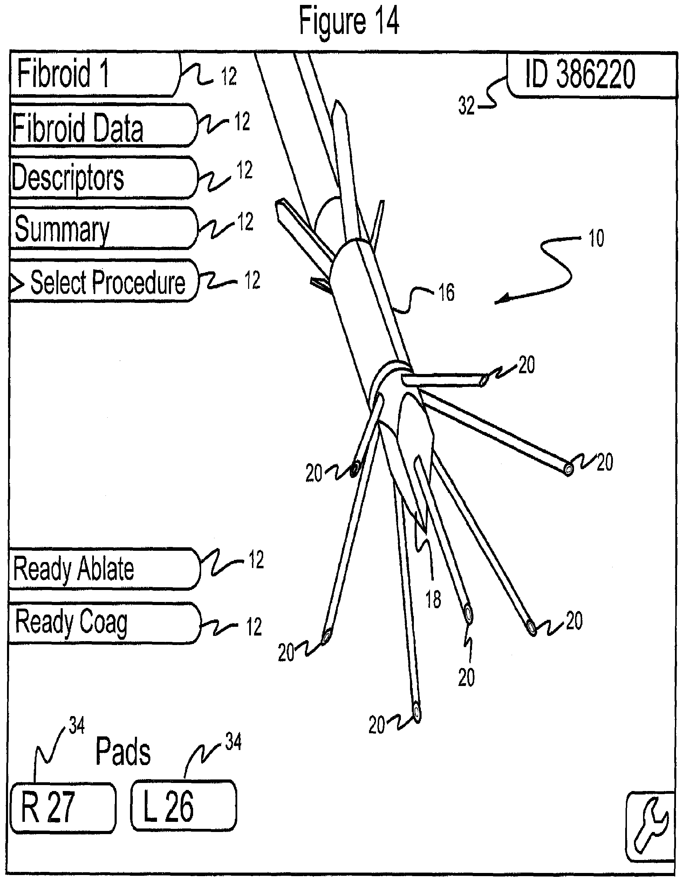

Referring to Fig. 14, the menu 12 choice "select procedure" is scrolled to by the practitioner to perform a surgical procedure of ablating a fibroid tumor. The practitioner can choose to bypass the "fibroid data", "descriptors" and "summary" menus 12 and scroll directly to the "select procedure" menu 12.

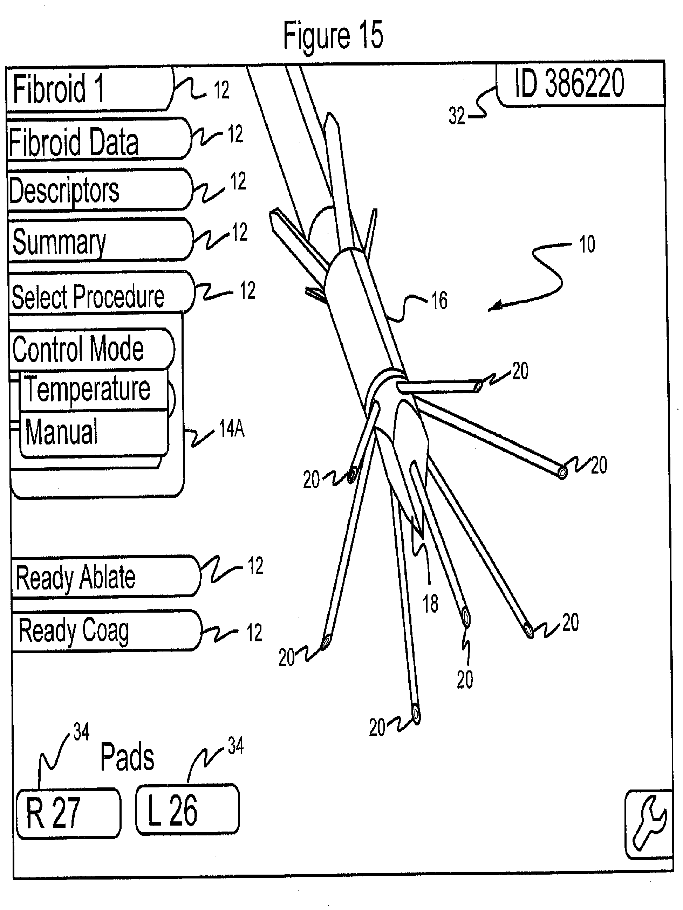

Referring to Figs. 15-18, by selecting the "select procedure" menu 12, a "control mode" submenu 14 is displayed for selecting a target temperature and a target power level for RF ablation energy to be sent to trocar 18 and each of seven stylets 20 (RF energy delivery electrodes in accordance with the preferred embodiment) of the ablation device 16. Stylets 20 are illustrated on the touch screen display, which is itself illustrated in, for example, Figs. 15-18. Stylets 20 have an internal volume that contains a wire thermocouple transducer, which performs the function of measuring temperature of the target tissue during the procedure which allows control of the ablation operation and ensures that the target tissue will become necrotic.

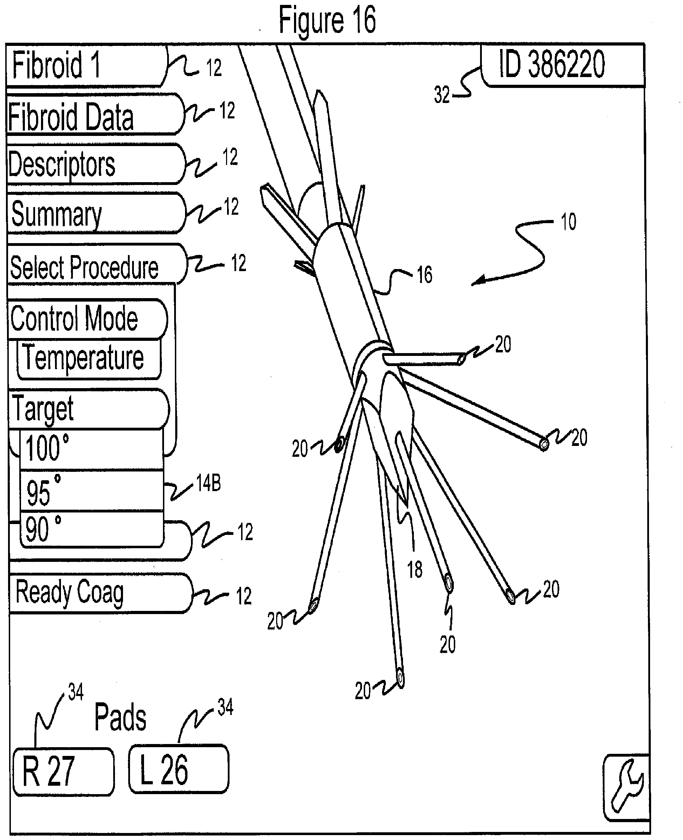

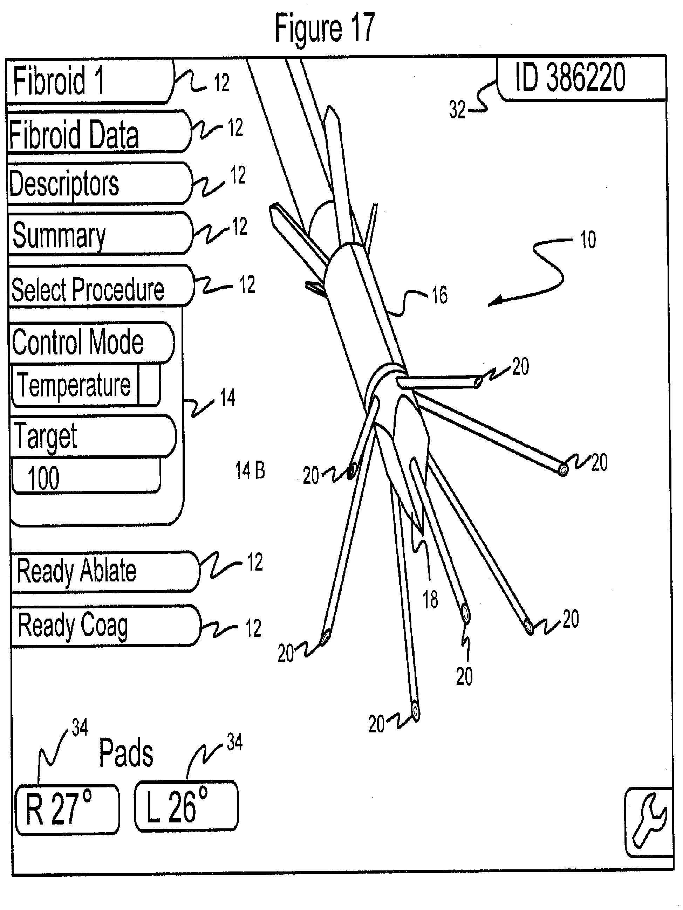

When submenu 14 item "control mode" is scrolled to and selected, "temperature" and "manual" submenus 14A are displayed (Fig. 15). The practitioner may then select the target temperature by selecting submenu 14A item "temperature". A further submenu 14B "target" is displayed having three temperature choices: 100° C, 95° C and 90° C (Fig. 16), from which the practitioner can select a target temperature. Fig. 17 shows the display upon selection of 100° C by the practitioner as the target temperature chosen for stylets 20 of the ablation device 16. In this mode the system controls the temperature to be at approximately the selected temperature, in the example 100 degrees C by turning RF energy on and off, for as long as the foot pedal coupled to the RF generator is telling the system to stay on.

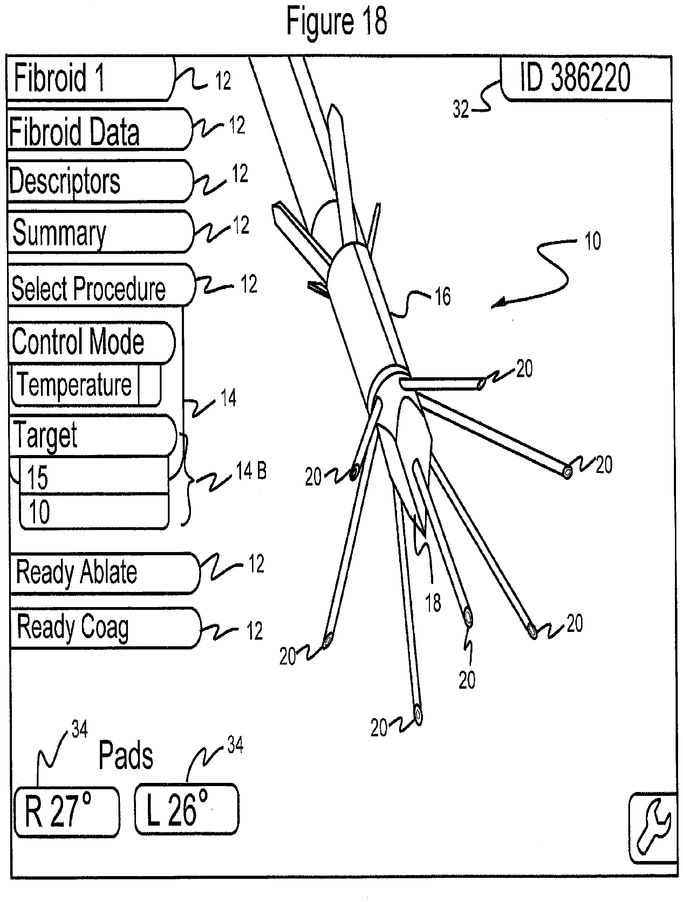

Alternatively, the surgeon may scroll to and select" Manual" from the menu as illustrated in the screen of Figure 15. This brings up the screen of Figure 18. The practitioner chooses the target power level of RF energy emitted from the seven stylets 20 by selecting the desired power from further submenu 14B, where "target" is displayed. A choice of two target power levels is presented, namely 15 watts and 10 watts (Fig. 18), from which the practitioner can select the target power level. In this mode the surgeon or other practitioner uses the foot pedal to "manually" turn the RF output on and off.

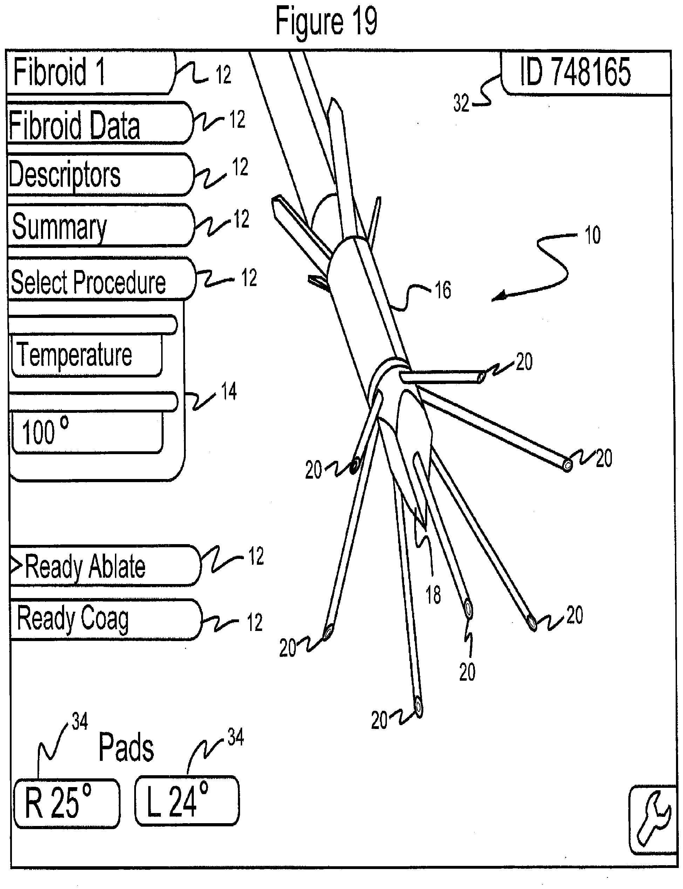

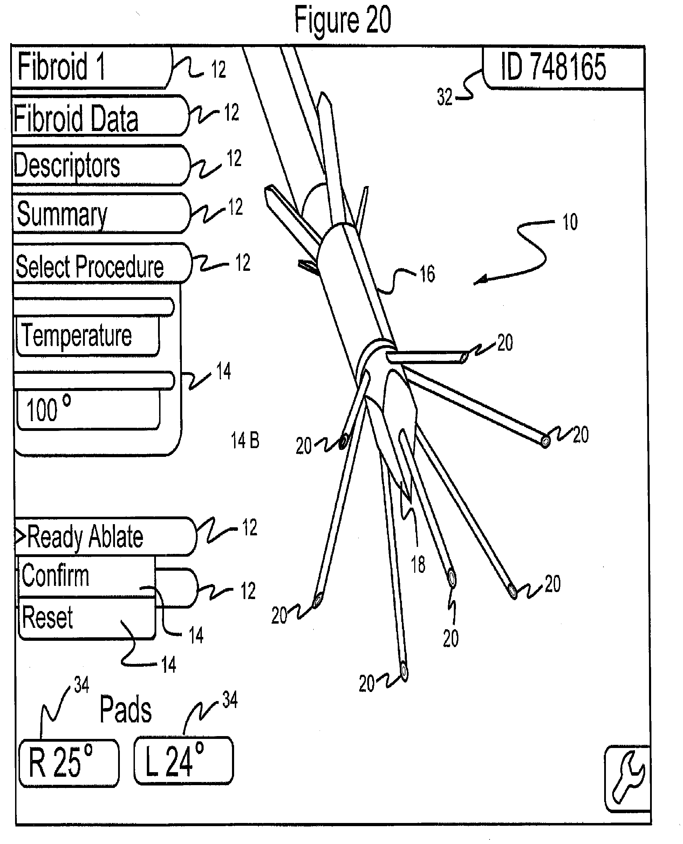

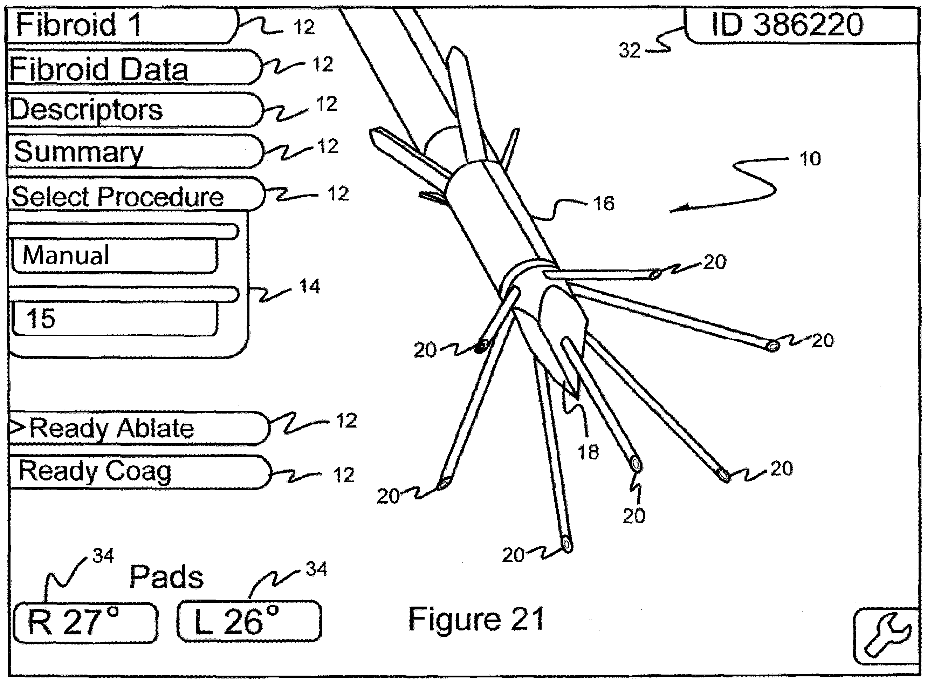

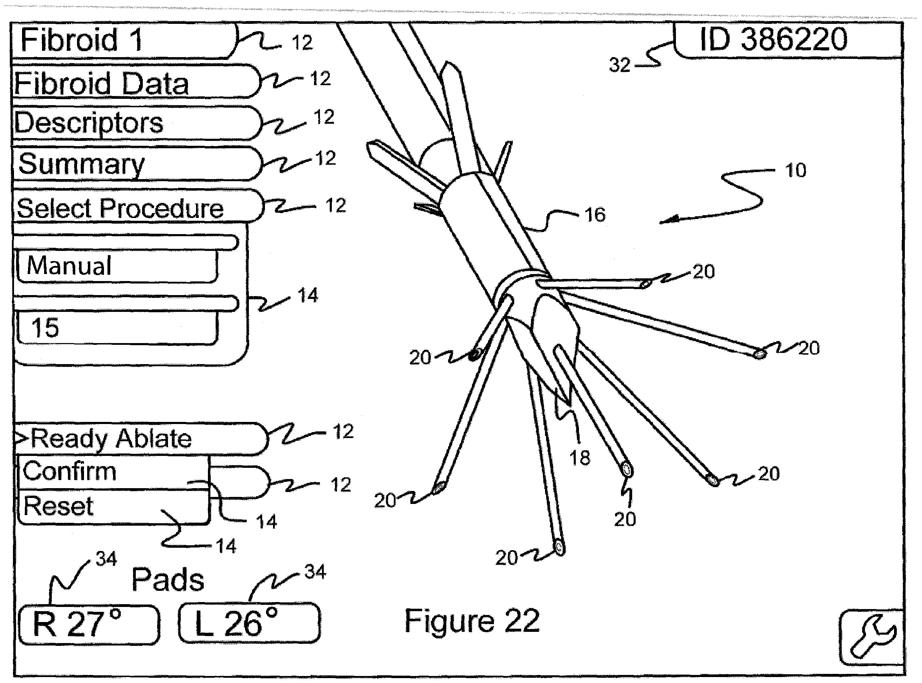

Returning back to the case where the surgeon has elected to have the system maintain a target temperature automatically by turning the RF energy on and off in response to temperature measurements by the transducers in the stylets, and referring to Figs. 19-22, the practitioner starts ablation of the fibroid tumor by scrolling to and selecting the menu 12 "ready ablate" (Figs. 19 and 21), which displays two submenu 14 items, namely "confirm" and "reset" (Figs. 20 and 22). "Reset" brings the system back to the screen of Figure 15, for selection between temperature and manual control.

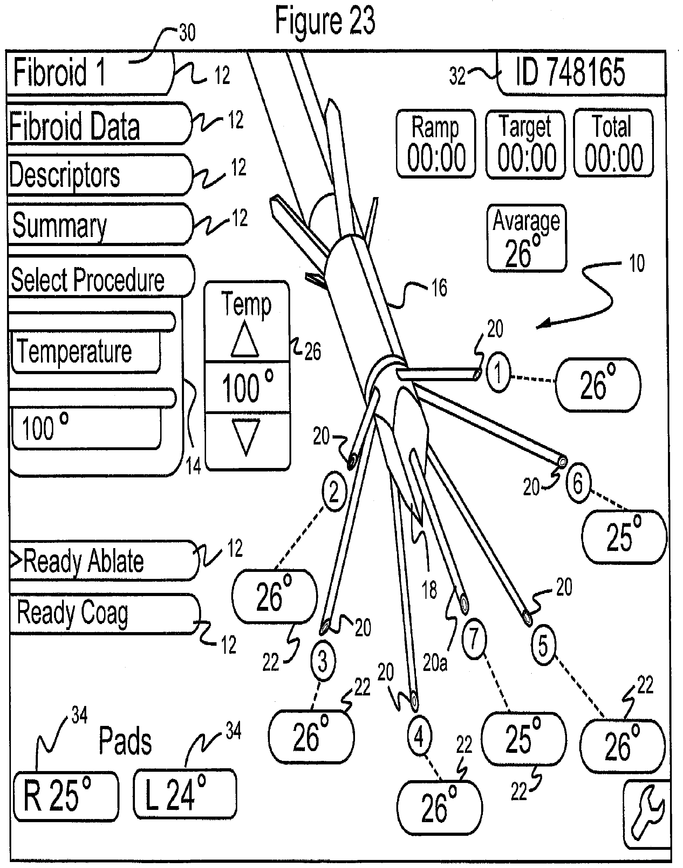

In accordance with a preferred embodiment, and referring to Figs. 23-27, after selecting the target temperature 26, the menu 12 "ready ablate", and the submenu 14 item "confirm", a new screen (Fig. 23) is displayed on the GUI 10 which shows the ablation device 16 and various parameters of the surgical field . The practitioner can scroll through this new screen and make adjustments to the target temperature 26 (for example in 1 degree C increments) by selecting the select button on the navigational tool. Likewise, if manual control has been selected, adjustments to the selected power level 30 can be made, for example, in one watt increments. The practitioner can return to the main menu by scrolling through the menu 12 choices and selecting any menu 12 item other than "ready ablate" or "ready coag" in, for example, the screen of Figure 23.

In the preferred embodiment, the number assigned to the fibroid undergoing ablation, i.e., "fibroid number" indication 36, is displayed. The number assigned to the patient, i.e., patient "ID" number 32, is also displayed. The target temperature indicator 26 set by the practitioner is displayed. The target temperature 26 may be adjusted up or down by the practitioner during the surgical procedure. Stylet temperature readings 22 for each of the seven stylets 20 are displayed. Average temperature display 24 which reads the average temperature in the tissue adjacent all of the stylets 20 is displayed. The practitioner may choose to not use certain stylets 20 as part of the calculation of the average temperature 24. For example, the highest and lowest measurement readings may be removed from the calculation. Typically, the central stylet (stylet 20a) is not used in the calculation of the average temperature 24 as this stylet tends to be mechanically deflected in the tissue mass in a difficult to predict manner. The temperature of the right and left pads is displayed by indicator 34. Such pads contain three thermocouples. Display 34 indicates the highest temperature on each pad, and is displayed for the right and left pads, displayed in the indicators labeled "R" and "L" on GUI 10. The time elapsed from the start of the application of RF ablation energy is displayed as "ramp" time on display 27 on GUI 10. The time elapsed once the temperature of the tissue mass has reached the preset target temperature displayed on indicator 26 (which is the average temperature displayed in indicator 24 in tissue adjacent to each of the seven stylets 20 selected for this calculation) is displayed as "target" time on indicator 28 on GUI 10. The target time (that is time at the targeted temperature) displayed on indicator 28 at this preset target temperature shown on indicator 26 can range from about 10 seconds to about 20 minutes depending on the size of the tissue mass being ablated and deployment of the ablation device. The total time elapsed from the start of ablation to the end of ablation is displayed as "total" time indicated on display 29 on GUI 10.

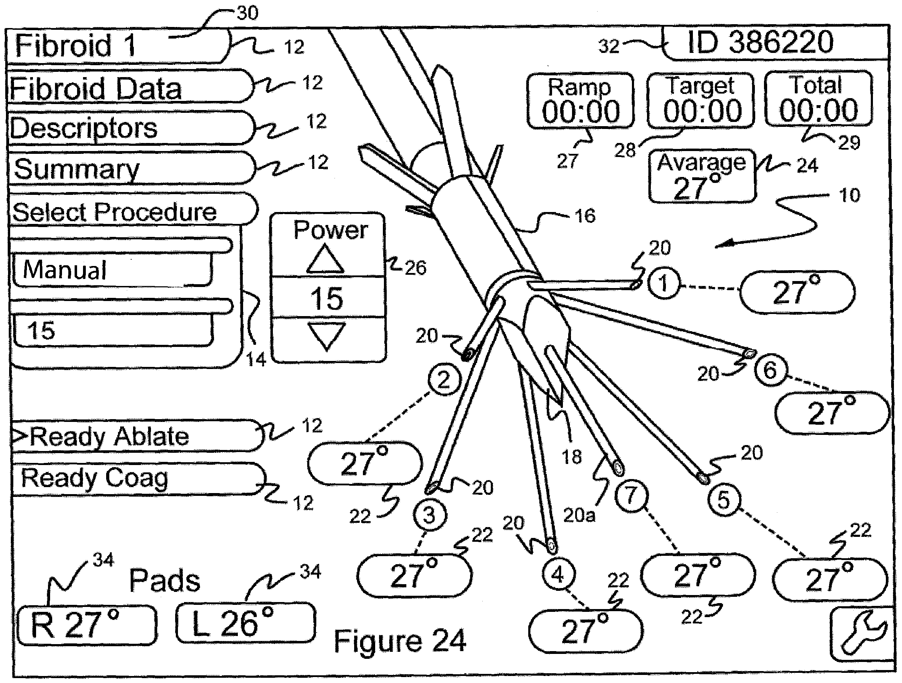

Referring to Fig. 24, operation in the "manual" mode is similar. After selecting the target power level indicated on display 30, the menu 12 item "ready ablate", and the submenu 14 "confirm" (Figure 22), GUI 10 displays the same parameters as described hereinabove with the exception that the target power level (indicated on display 30) chosen by the practitioner. In the example shown in Fig. 24, this is 15 watts. The target power level indicated in display 30 may be adjusted up or down by the practitioner during the surgical procedure. The target power level indicated in display 30 represents the amount of RF energy emitted from the stylets and trocar of the ablation device at a standard frequency of 460 KHz.

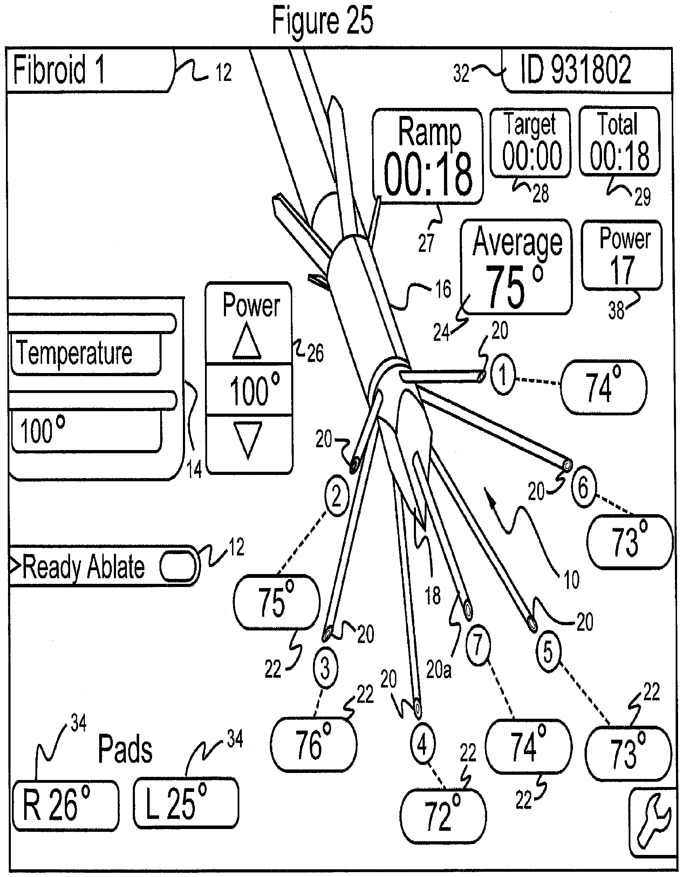

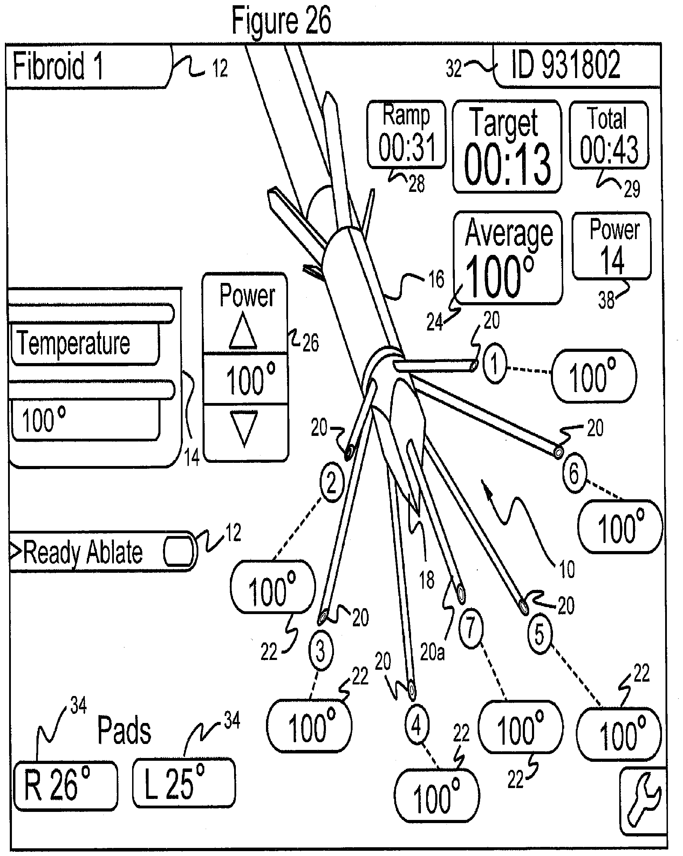

In accordance with the invention, the practitioner starts the ablation procedure by depressing and releasing a foot pedal. This activates the ablation device 16 to begin emission of radiofrequency energy through the stylets 20 into the site of the tissue mass. Fig. 25 shows that with the target temperature indicated on display 26 set at 100° C and 18 seconds into the ablation procedure, as indicated by the "ramp" time display 27, six of the stylets show an average temperature indicated on display 24 of 75° C, the right and left pads show temperatures on display 34 of 26° C and 25° C, respectively, and the power is indicated on display 38 at 17 watts. Fig. 26 shows that with the target temperature 26 set at 100° C and 31 seconds into the ablation procedure, as indicated by the "ramp" time on display 27, and 13 seconds after reaching the target temperature indicated on display 26, as indicated by the "target" time display 28, for a "total" time indicated on display 29 of 43 seconds, all six of the stylets show that the target temperature 24 of 100° C has been reached, the right and left pads 34 show temperatures of 26° C and 25° C, respectively, and the power level 38 has decreased to 14 watts.

When the target temperature indicated on display 26 is reached, the power level 38 decreases. In accordance with the invention, there is provided a high power safety limit that shuts off the power if the target temperature 26 has not been reached in 1.5 minutes into the ablation procedure and the power has increased up to 200 watts. In addition, as another safety limit, it is provided that when the temperature of the three thermocouples in the right and left pads on the thighs of the patient reach 40° C, the pad temperature displays 34 turn yellow on the GUI. At 43° C, they turn red and at 44° C radiofrequency emission is shut off.

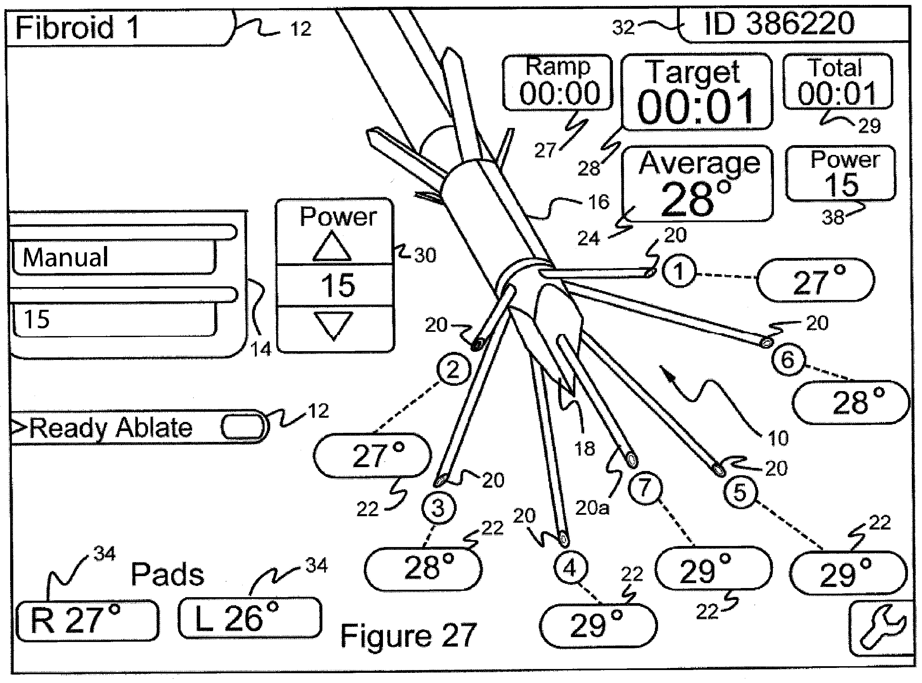

Fig. 27 shows the operation of the ablation device using the menu 12 choice "manual" and presetting a target power level on display 30 at 15 watts, in which after one second, as indicated by the "ramp" time 27, the power level 38 has reached the target power level 30 of 15 watts.

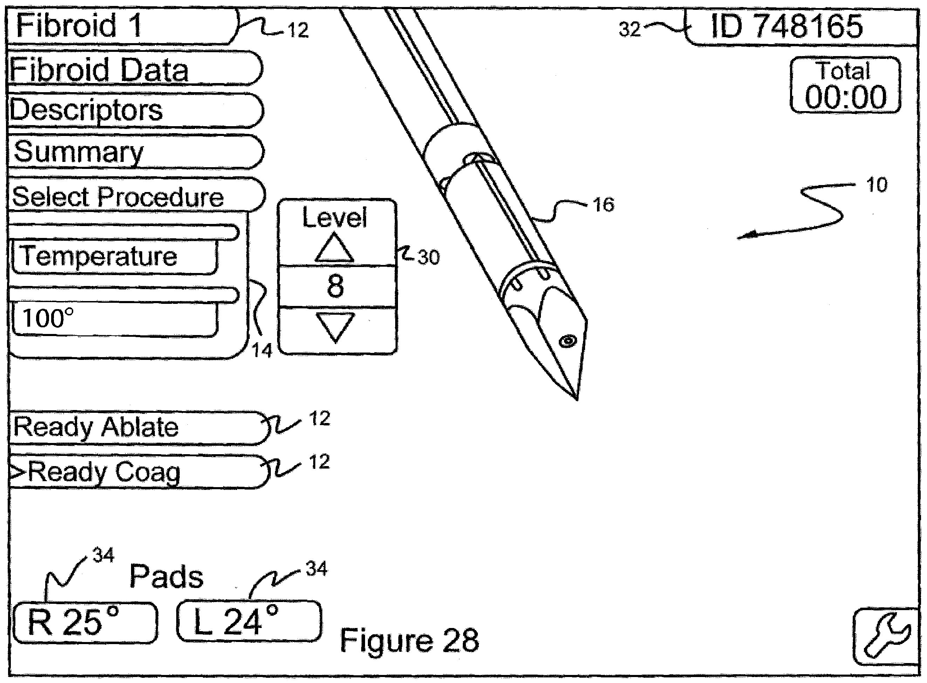

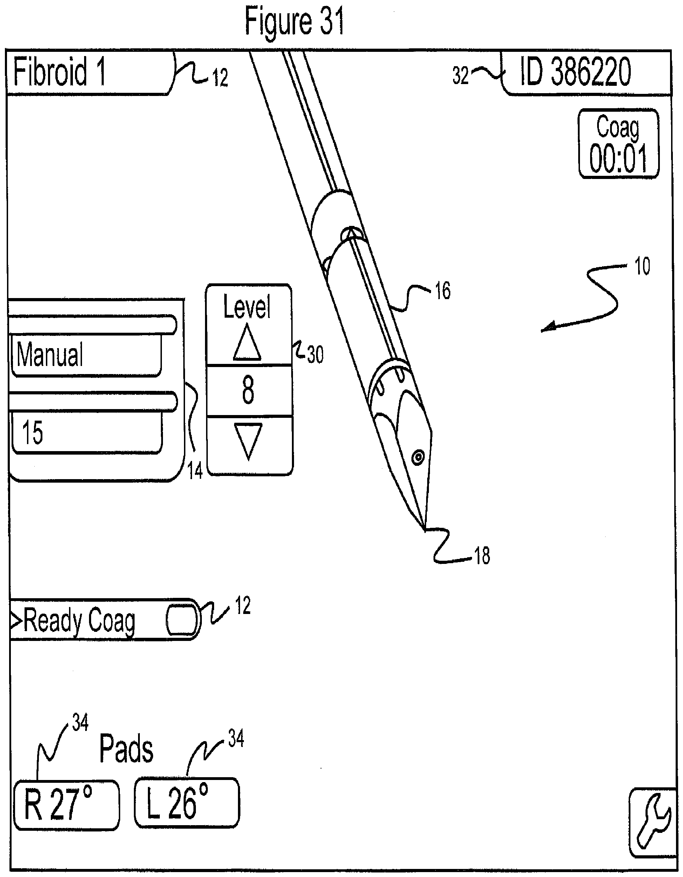

In accordance with a preferred embodiment, and referring to Figs. 28-31, coagulation of the track followed by the trocar to the uterine fibroid is achieved by selecting the menu 12 item "ready coag" and pressing and holding the foot pedal for the duration of the withdrawal of the trocar and coagulation of the path of entry of the trocar to the uterine fibroid. The coagulation procedure is performed by RF energy emitted from the trocar 18 of the ablation device 16 in which the stylets and anchors of the ablation device 16 have been retracted. Fig. 28 shows an illustration of the ablation device 16 with retracted stylets on GUI 10, a preset target temperature indicated on display 26 at 100° C, a target power level indicated on display 30 at 8 watts, and right and left pad temperatures of 25° C and 24° C, respectively, indicated on the display 34.

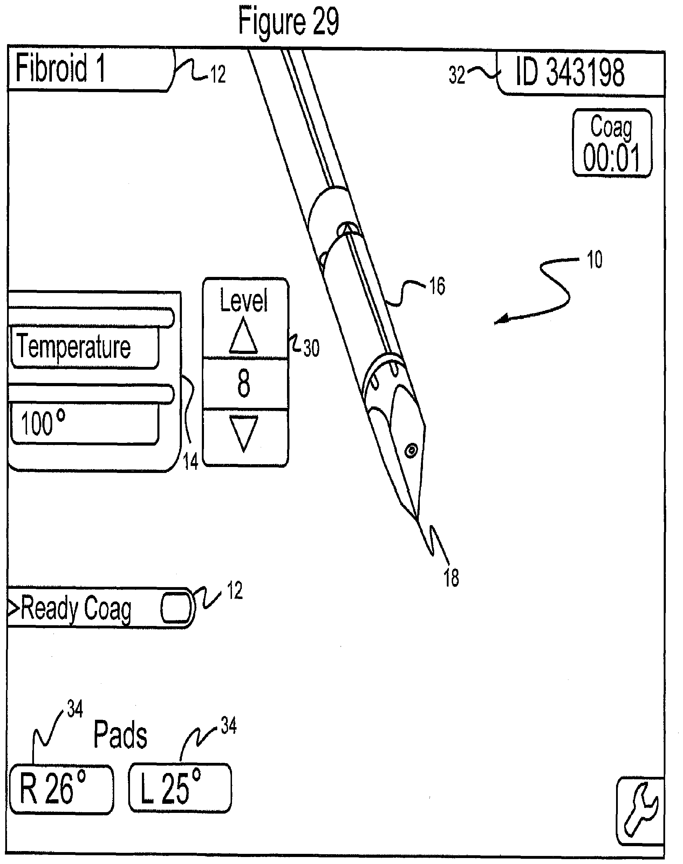

After selecting "ready coag" the screen of Figure 29 appears. Fig. 29 shows that the temperature in the right and left pads 34 has risen to 26° C and 25° C, respectively, one second into coagulation. Here coagulation is done at a temperature of 100°C.

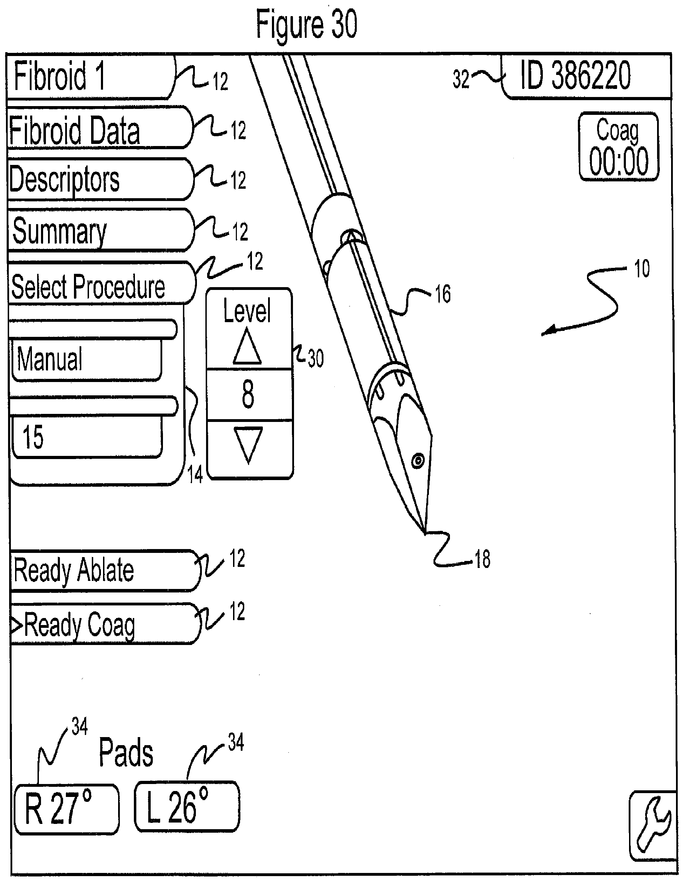

Coagulation may also be done in the "Manual" mode with the power set to 15 W, as illustrated in Figure 30. Fig. 30 shows the operation of the ablation device using the menu 12 choice "manual" and a preset target power level 30 rather than a preset target temperature. The target power level on indicator 30 is set to 8 watts prior to the start of coagulation of the trocar track ("coag" time: 0 seconds) and the temperature of the right and left pads, indicated on display 34, is 27° C and 26° C, respectively.

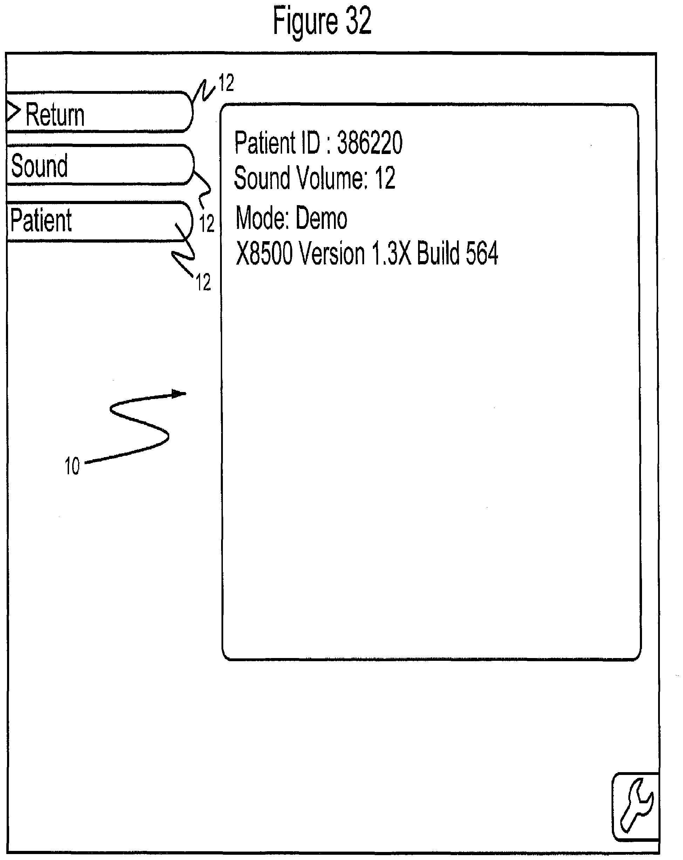

In accordance with the invention and referring to Figs. 32-40, when the surgical procedure is completed, the practitioner can select the menu 12 "return" item, which displays on the GUI 10 patient identification number and various features of the computer software program, such as sound volume, software mode and software version (Fig. 32). In addition, the computer software program contains menu choices for keeping patient records and allows for the selection of text in different languages. Notes may also be kept by dictation in different "sound volumes" associated with the patient.

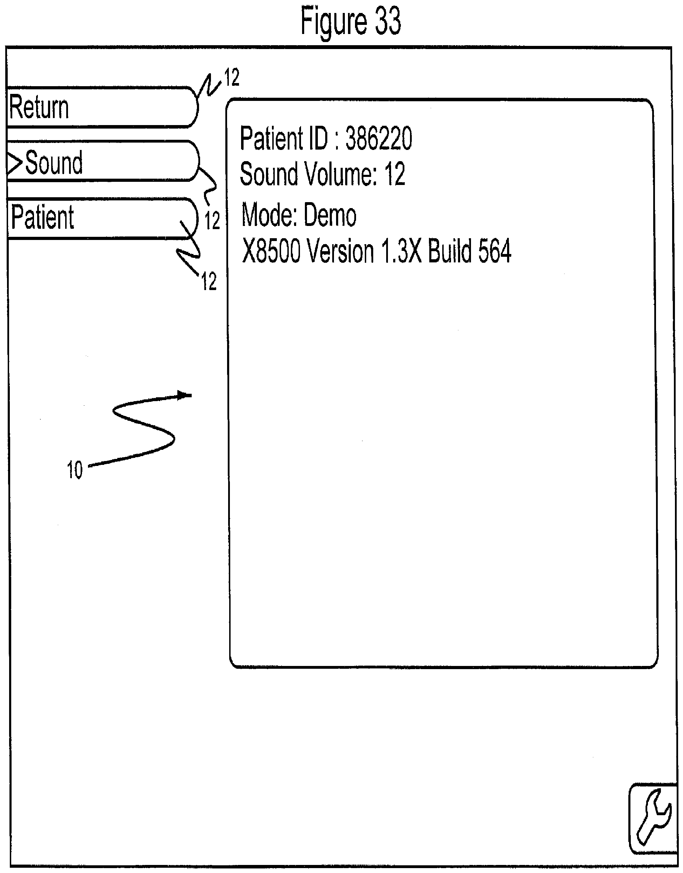

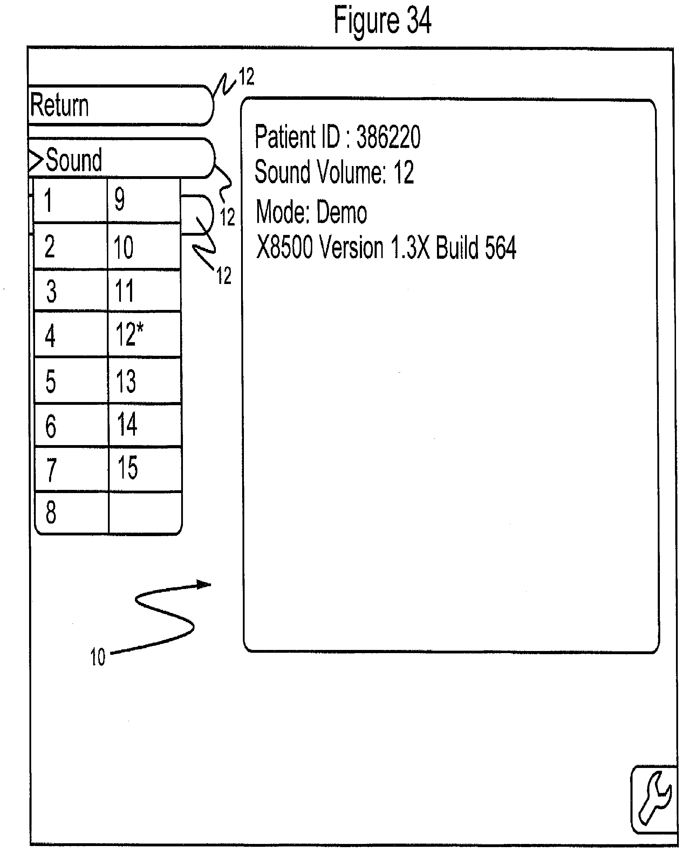

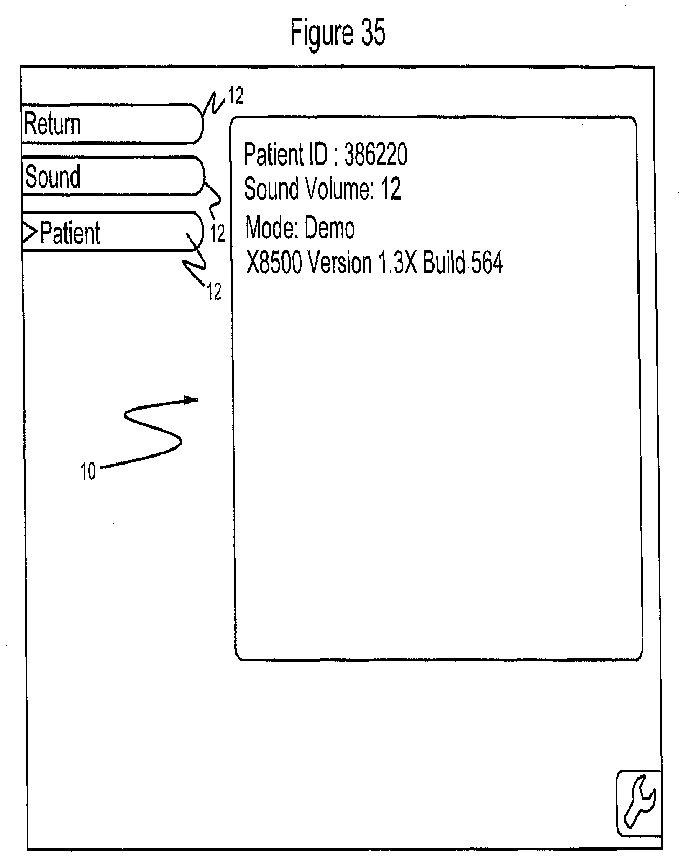

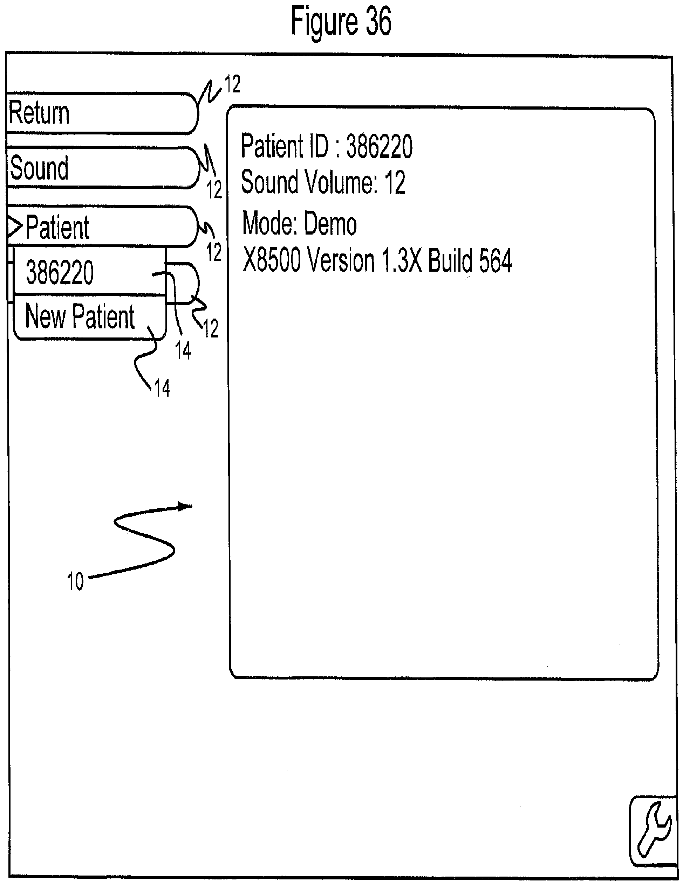

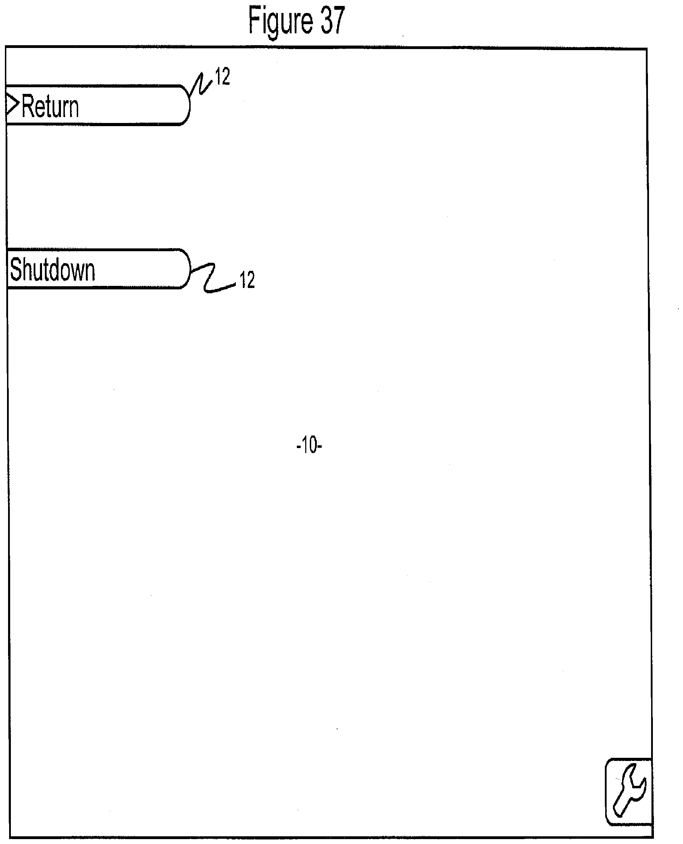

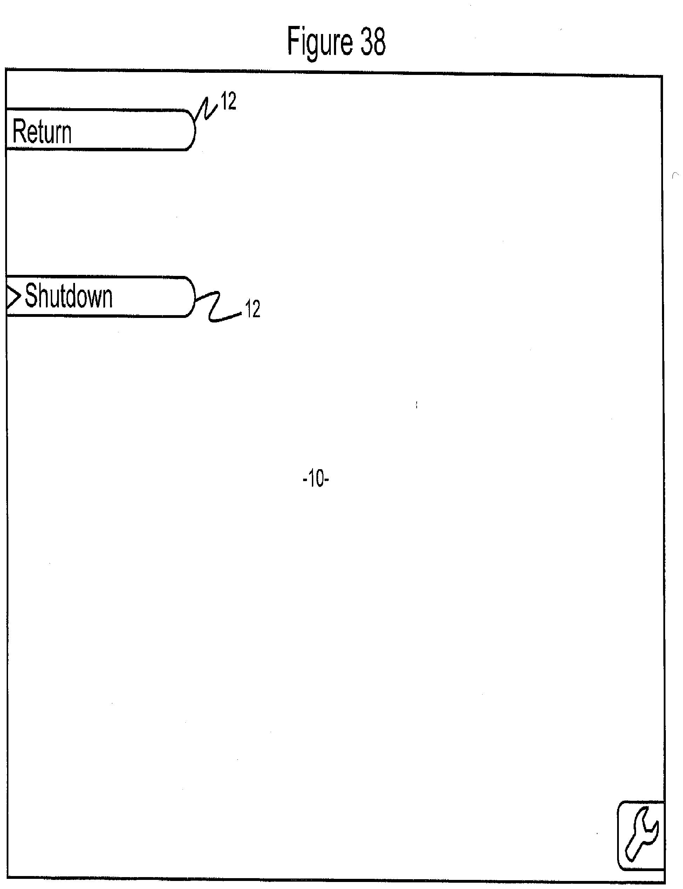

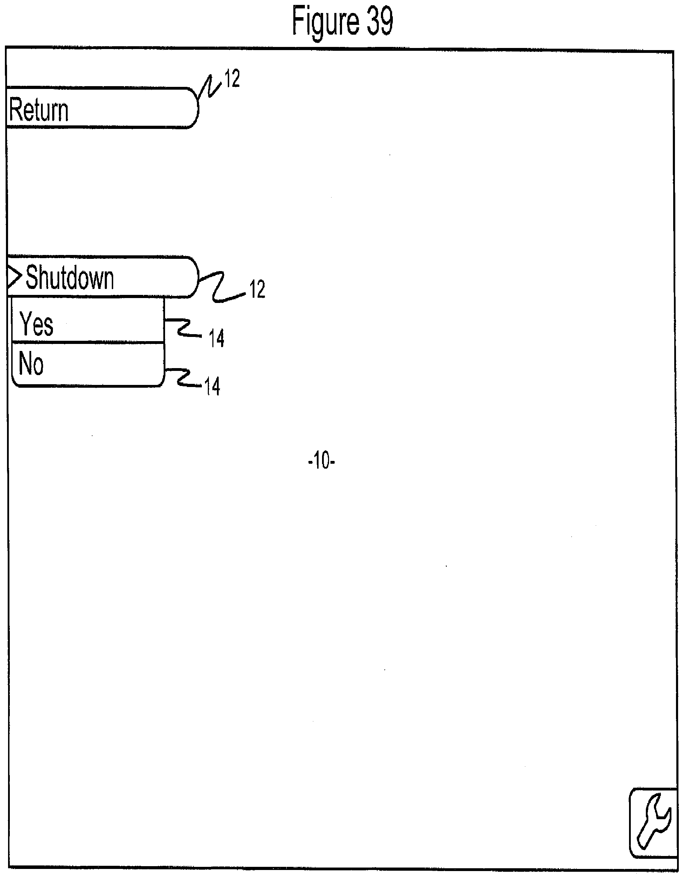

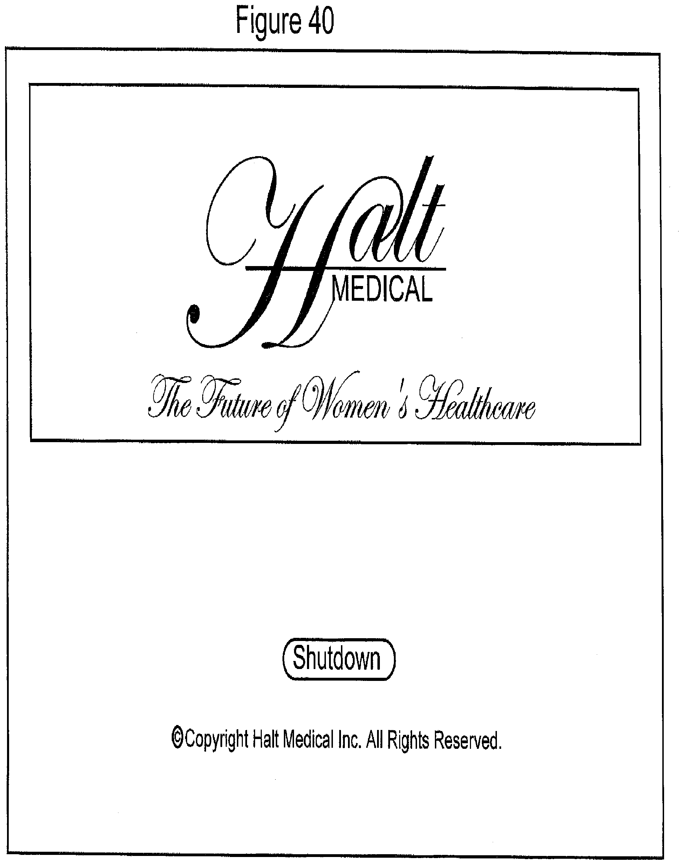

For example, by selecting the "sound" menu item 12 (Fig. 33), a series of sound volumes are shown from which the practitioner can select. Fig. 34 shows the selection of sound volume 12. By selecting the "patient" menu 12 (Fig. 35), the practitioner can select a patient identification number or enter a new patient identification number (Fig. 36). By selecting the "return" menu 12 again (Fig. 37), the practitioner can scroll to a "shutdown" menu 12 (Fig. 38), select the "shutdown" menu 12, and then select from the submenus 14 "yes" or "no" to shut down the computer program (Fig. 39). Fig. 40 shows the GUI after the computer program has been shut down.

An exemplary system for implementing the above invention is illustrated in Figure 1b. Generally, the system 110 comprises a computer 112. Computer 112 may be any control device, such as a microprocessor, personal computer or a more powerful or less powerful computer with a typical personal computer-type operating system. Computer 112 includes a display screen 114, which may optionally be a touchscreen to provide a second means of navigation.

Personal computer 112 also incorporates software 116. Software 116 may be of any type for use on any suitable computing device, and which may be easily written by a programmer of ordinary skill in the art who is informed by this specification. The software is responsive to produce images illustrated in the drawings and stored in a memory 118 of computer 112. The software performs the navigation functions described above, being responsive to touchscreen entry and/or scroll and select buttons 23 and 25 on ablation instrument 1.

Computer 112 communicates with ablation instrument 1 through an interface board 120 which is coupled to scroll and select buttons 23 and 25. Likewise, in response to operation by touching on display screen 114 or operation of scroll and select buttons 23 and 25, computer 112 may cause RF generator 122 to apply power to the trocar point for ablation. In response thereto, thermocouples on stylets 20 will generate temperature indicating signals which are coupled through suitable interface electronics to computer 112, allowing the computer to control application of RF generator by RF generator 122, to display temperature information, operate alarms, to terminate the application of RF energy, and to perform any other design controls in response thereto, for example as described above.

In accordance with United States Patent No. 6,840,935 issued to Lee on January 11, 2005, uterine ablation may be implemented with imaging provided through the use of a laparoscope imaging arrangement and an ultrasound imaging device. The images generated by the laparoscope and the ultrasound device are provided on separate monitors.

In accordance with the present invention, it is contemplated that the display of the present invention, as detailed above, may include touchscreen controls and/or menu options for controlling other devices. For example, the display may provide for navigation to a control menu for controlling display characteristics for the ultrasound viewing device, a control menu for selecting metering functions for inclusion on the display, such as heartbeat, or for selection between ultrasound and laparoscopic images.

The inventive system may also incorporate means for varying the various menu functions described above incorporated into the software which controls the system. Such means may comprise accessing menu choices and display options using a keyboard.

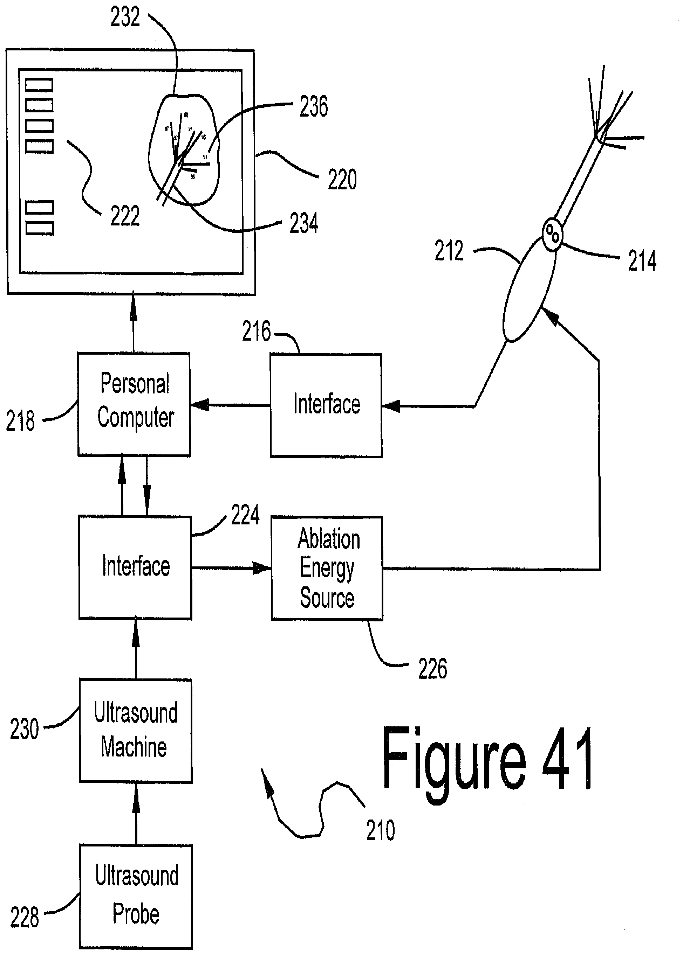

In accordance with a particularly preferred embodiment of the present invention, the display of menu options (and the other GUI elements, or some of them) as detailed in Figures 1-40, may also be incorporated into the display of, for example, the ultrasound image used by the physician. Other types off images may also be employed. More particularly, with reference to Figure 41, the inventive system 210 utilizes an ablation probe 212. Ablation probe 212 includes a multi-button keypad 214, for example with scroll and select switches.

In the manner of the earlier embodiment, temperature signals and keypad control information is coupled to a computer interface 216 which sends this information to personal computer 218. Personal computer 218 drives a computer display 220 which includes a navigation menu 222 of the type described above in connection with Figures 1-40.

As detailed above in connection with Figures 1-40, personal computer 218 through interface board 224 controls ablation energy source 226. At the same time, an ultrasound probe 228 coupled to an ultrasound machine 230 provides ultrasound image information to interface 224 which in turn provides this information to personal computer 218 for display on computer display 220.

Using the system of Figure 41, the surgeon may concentrate on a single monitor displaying both ultrasound, and device performance information and a means for control of the system. More particularly, computer display 220 displays, for example, the fibroid 232 being operated on, an image 234 of probe 214 and an image 236 of temperature data. The positioning of the images 234 and 236 may be done by the computer using a pattern matching or other strategy.

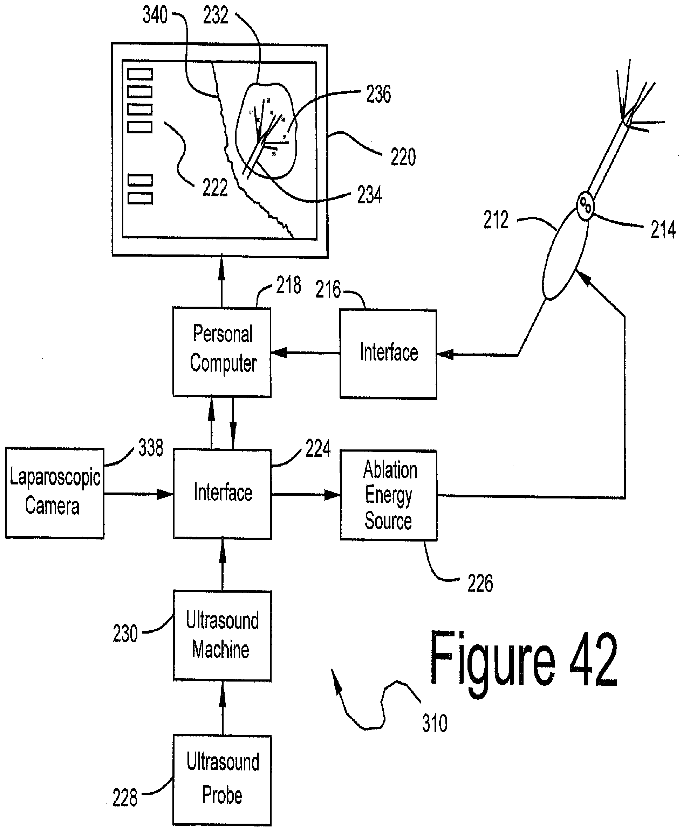

A most preferred embodiment of the present invention is illustrated in Figure 42. The operation of the system 310 of Figure 42 is substantially the same as that of the system in Figure 41, except for the addition and integration of an image from a laparoscope.

More particularly, a laparoscopic camera 338 is coupled to interface 224. Camera 338 produces an image of the outside of the uterus resulting in display of an image 340 of the uterus on computer display 220 superimposed over the image 232 of the fibroid obtained using ultrasound. It is noted that images 232 and 340 are positioned in the same manner as the fibroid and the uterus are positioned in the patient, thus giving a more complete picture of the state of the surgery.

In accordance with the invention, the display on the embodiment of Figure 42 may display images of the ablation probe that are synthesized based on probe angular orientation, probe position, probe size, ablation stylet deployment and probe structural configuration, or similar or equivalent information. In addition, reticle or other guidance indicators may be shown on the display. Probe angular orientation and probe position may be detected using prior art position detection systems tha, for example, utilize laser, optical, rf, ultrasound, electromagnetic or other position detection systems.

It will be appreciated by those skilled in the art that changes can be made to the embodiments described above without departing from the broad inventive concept thereof. It is understood, therefore, that this invention is not limited to the particular embodiments disclosed, but it is intended to cover modifications that are within the scope of the invention, as defined by the appended claims.

## Claims

1. A system for remote control of a radio frequency ablation surgical device, the system comprising:
(a) a first imaging device (228) of a first type having a first image output (232), said first imaging device (228) being positioned to image an area being subject to surgery;
(b) a second imaging device (338) of a second type having a second image output (340) said second imaging device (338) being positioned to image said area being subject to surgery;
(c) a computer (112) coupled to receive said first (232) and second (340) image outputs and merge said first (232) and second (340) image outputs into a unitary image output representing a unitary image;
(d) a computer software program (116), resident in said computer (112) for generating a graphic user interface (10) including a menu (12) and submenu (14) items;
(e) a radiofrequency ablation surgical device (16) coupled to said computer (112), said ablation device (16) comprised of an operator handle (3) on one end, and a trocar (5) and a plurality of stylets (20) on an opposite end;
(f) a navigational button matrix comprised of a two button electronic control system mounted on the operator handle (3) of the ablation device (16), said two button electronic control system comprised of a scroll button (23) and a select button (25) that interacts with the computer software (116) displayed on the graphic user interface (10);
(g) software (116), resident in said computer (112), for receiving and displaying information received from said surgical device (16) and/ or for controlling the operation of said surgical device (16); and
(h) a display coupled to said computer (112) for displaying said graphic user interface (10) and said unitary image;
(i) the trocar (5), the plurality of stylets (20) and the two button electronic control system with scroll button (23) and select button (25), each of said plurality of stylets (20) having an internal volume that contains a wire thermocouple transducer for measuring temperature, said plurality of stylets (20) conducting radiofrequency radiation from a radiofrequency source (226), wherein an electrical current travels from the stylets (20) of the ablation device (16) through a tissue mass of the patient and to a right and a left pad (34) placed atop the right and the left leg, respectively, of the patient, the highest temperature reached by the tissue mass adjacent to the stylets (20) of the ablation device (16) in each of the right and the left pads (34) being displayed on the graphical user interface (10) as a right and a left pad temperature component, wherein depression of the scroll button (23) moves through the menu items (12) associated with the submenu items(14) displayed on the graphic user interface, and depression of the select button (25) selects a desired menu item (12) or submenu item (14);
(j) wherein the first imaging device (228) comprises an ultrasound probe having an ultrasound image output and which is configured to create an image of the area being subject to surgery such that the ultrasound image output of an internal region of the area is displayed in relation to an image representative of the ablation surgical device and a graphical user interface;
(k) wherein the second imaging device (338) comprises a laparoscopic camera having a laparoscope image output which is configured to be positioned to create an image of a region of said area being subject to surgery; and
(l) wherein the computer (112) is coupled to receive and merge said ultrasound image output and said laparoscope image output into the unitary image for display on said graphical user interface display such that the ultrasound image output and laparoscope image output are superimposed over one another.

2. The system of claim 1, further comprising depressing a foot pedal by the practitioner to send radio frequency radiation through the trocar (5) and/or plurality of stylets (20) of the ablation device (16) in order to ablate or coagulate the tissue mass of the patient.

3. The system of claim 2, wherein the trocar (5) and the plurality of stylets are deployed adjacent to the tissue mass in the patient.

4. The system of claim 1, wherein the series of menus (12) are selected from the group consisting of fibroid data, descriptors, summary, select procedure, ready ablate and ready coag choices.

5. The system of claim 4, wherein the fibroid data menu includes submenu (14) choices selected from the group consisting of number (14a), diameter (14b), position (14c) and type (14d).

6. The system of claim 5, wherein the position choices (14c) are selected from the group consisting of anterior, posterior and lateral positions (14C).

7. The system of claim 6, wherein the anterior, posterior and lateral position (14C) choices each include submenus selected from the group consisting of midline, right and left choices (14D).

8. The system of claim 7, wherein the submenu for midline, right and left choices (14D) each include a further submenu selected from the group consisting of fundal, miduterine, lower segment and cervical choices.

9. The system of claim 8, wherein the submenu for fundal, mid-uterine, lower segment and cervical choices (14E) each include a further submenu selected from the group consisting of intramural, subserosal, submucous land submucous 2 choices (14F).

10. The system of claim 4, wherein the select procedure menu (12) includes the submenus (14) selected from the group consisting of temperature, manual and impedance choices.

11. The system of claim 4, wherein ablation of the tissue mass by the ablation device (16) is achieved by selecting the ready ablate menu (12) and a confirm submenu (14), pressing and releasing the foot pedal to start ablation of the tissue mass and pressing and releasing the foot pedal to stop ablation of the tissue mass.

12. The system of claim 1, wherein the ablation of the tissue mass is effected by emission of radio frequency energy from each of the stylets (20) which causes temperature in the tissue mass adjacent to the stylets to increase to a preset target temperature (26) as measured by the plurality of stylets (20).

13. The system of claim 12, wherein each of the plurality of stylets (20) displays on the graphical user interface (10) the temperature of the tissue mass adjacent to each of the plurality of stylets (20), and wherein an average temperature (24) of the tissue mass is calculated from the temperatures indicated by two or more of the plurality of stylets (20) adjacent to the tissue mass, said average temperature (24) displayed on the graphical user interface (10).

## Patentansprüche

1. System zur Fernsteuerung einer chirurgischen Radiofrequenz-Ablationsvorrichtung, wobei das System umfasst:
(a) eine erste Bildgebungsvorrichtung (228) eines ersten Typs mit einer ersten Bildausgabe (232), wobei die erste Bildgebungsvorrichtung (228) positioniert wird, um einen Bereich abzubilden, der einer Operation unterzogen wird;
(b) eine zweite Bildgebungsvorrichtung (338) eines zweiten Typs mit einer zweiten Bildausgabe (340), wobei die zweite Bildgebungsvorrichtung (338) positioniert wird, um den Bereich abzubilden, der einer Operation unterzogen wird;
(c) einen Computer (112), der verbunden ist, um die erste (232) und die zweite (340) Bildausgabe zu empfangen und die erste (232) und die zweite (340) Bildausgabe zu einer einheitlichen Bildausgabe zusammenzuführen, die ein einheitliches Bild darstellt;
(d) ein Computersoftwareprogramm (116), das auf dem Computer (112) vorhanden ist, um eine graphische Benutzerschnittstelle (10) zu erzeugen, die ein Menü (12) und Untermenüpunkte (14) enthält;
(e) eine chirurgische Radiofrequenz-Ablationsvorrichtung (16), die mit dem Computer (112) verbunden ist, wobei die Ablationsvorrichtung (16) einen Bedienerhandgriff (3) an einem Ende und einen Trokar (5) und eine Vielzahl von Stylets (20) an einem entgegengesetzten Ende umfasst;
(f) eine Navigationstastenmatrix, umfassend ein elektronisches Steuersystem mit zwei Tasten, das auf dem Bedienerhandgriff (3) der Ablationsvorrichtung (16) angebracht ist, wobei das elektronische Steuersystem mit zwei Tasten eine Rolltaste (23) und eine Auswahltaste (25) umfasst, die mit der auf der graphischen Benutzerschnittstelle (10) angezeigten Computersoftware (116) interagiert;
(g) Software (116), die auf dem Computer (112) vorhanden ist, zum Empfangen und Anzeigen von Informationen, die von der chirurgischen Vorrichtung (16) erhalten wurden und/oder zur Steuerung des Betriebs der chirurgischen Vorrichtung (16); und
(h) eine mit dem Computer (112) verbundene Anzeige zum Anzeigen der graphischen Benutzerschnittstelle (10) und des einheitlichen Bildes;
(i) den Trokar (5), die Vielzahl von Stylets (20) und das elektronische Steuersystem mit zwei Tasten mit Rolltaste (23) und Auswahltaste (25), wobei jeder der Vielzahl von Stylets (20) ein Innenvolumen aufweist, das einen Draht-Thermoelement-Wandler zum Messen der Temperatur enthält, wobei die Vielzahl von Stylets (20) Radiofrequenzstrahlung von einer Radiofrequenzquelle (226) leiten, wobei ein elektrischer Strom von den Stylets (20) der Ablationsvorrichtung (16) durch eine Gewebemasse des Patienten und zu einem rechten und einem linken Pad (34) fließt, die jeweils oben auf dem rechten und dem linken Bein des Patienten angeordnet sind, wobei die höchste Temperatur, die von der Gewebemasse erreicht wird, die an die Stylets (20) der Ablationsvorrichtung (16) in jedem der rechten und linken Pads (34) angrenzt, auf der graphischen Benutzerschnittstelle (10) als eine rechte und eine linke Pad-Temperaturkomponente angezeigt wird, wobei Drücken der Rolltaste (23) durch die Menüpunkte (12) bewegt, die den Untermenüpunkten (14) zugeordnet sind, die auf der graphischen Benutzerschnittstelle angezeigt werden, und Drücken der Auswahltaste (25) einen gewünschten Menüpunkt (12) oder Untermenüpunkt (14) auswählt;
(j) wobei die erste Bildgebungsvorrichtung (228) eine Ultraschallsonde mit einer Ultraschallbildausgabe umfasst, und ausgelegt ist, um ein Bild des Bereichs zu erzeugen, der einer Operation unterzogen wird, so dass die Ultraschallbildausgabe eines internen Teils des Bereichs in Bezug auf ein Bild angezeigt wird, das für die chirurgische Ablationsvorrichtung, und eine grafische Benutzeroberfläche repräsentativ ist;
(k) wobei die zweite Bildgebungsvorrichtung (338) eine laparoskopische Kamera mit einer laparoskopischen Bildausgabe umfasst, die ausgelegt ist, um so positioniert zu werden, dass sie ein Bild eines Teils des Bereichs erzeugt, der einer Operation unterzogen wird; und
(l) wobei der Computer (112) verbunden ist, um die Ultraschallbildausgabe und die laparoskopische Bildausgabe zu empfangen und in das einheitliche Bild zur Anzeige auf der graphischen Benutzerschnittstellenanzeige zusammenzuführen,
so dass sich die Ultraschallbildausgabe und die laparoskopische Bildausgabe gegenseitig überlagern.

2. System nach Anspruch 1, ferner umfassend das Drücken eines Fußpedals durch den Behandler, um Radiofrequenzstrahlung durch den Trokar (5) und/oder eine Vielzahl von Stylets (20) der Ablationsvorrichtung (16) zu senden, um die Gewebemasse des Patienten abzutragen oder zu koagulieren.

3. System nach Anspruch 2, wobei der Trokar (5) und die Vielzahl von Stylets angrenzend an die Gewebemasse im Patienten angebracht werden.

4. System nach Anspruch 1, wobei die Reihe der Menüs (12) ausgewählt sind aus der Gruppe bestehend aus Myom-Daten, Deskriptoren, Zusammenfassung, Auswahlverfahren, bereites Ablat und bereite Koagulationsauswahlen.

5. System nach Anspruch 4, wobei das Menü für Myom-Daten ein Untermenü (14) mit Auswahlmöglichkeiten enthält, die ausgewählt sind aus der Gruppe bestehend aus Zahl (14a), Durchmesser (14b), Position (14c) und Typ (14d).

6. System nach Anspruch 5, wobei die Positionsauswahlen (14c) ausgewählt sind aus der Gruppe bestehend aus anterioren, posterioren und lateralen Positionen (14C).

7. System nach Anspruch 6, wobei die anteriore, posteriore und laterale Positionsauswahlen (14C) jeweils Untermenüs enthalten, die ausgewählt sind aus der Gruppe bestehend aus Mittellinien-, Rechts- und Linksauswahlen (14D).

8. System nach Anspruch 7, wobei die Untermenüs für die Mittellinien-, Rechts- und Linksauswahlen (14D) jeweils ein weiteres Untermenü enthalten, das ausgewählt ist aus der Gruppe bestehend aus Fundus-, Miduterin-, Untersegment- und Zervikal-Auswahlen.

9. System nach Anspruch 8, wobei das Untermenü für die Fundus-, Miduterin-, Untersegment- und Zervikal- Auswahlen (14E) jeweils ein weiteres Untermenü enthält, das ausgewählt ist aus der Gruppe bestehend aus intramuralen, subserosalen, submukösen land-submukösen 2 Auswahlmöglichkeiten (14F).

10. System nach Anspruch 4, wobei das Auswahlverfahren-Menü (12) die Untermenüs (14) enthält, die ausgewählt sind aus der Gruppe bestehend aus Temperatur-, Hand- und Impedanzauswahlen.

11. System nach Anspruch 4, wobei die Ablation der Gewebemasse durch die Ablationsvorrichtung (16) durch Auswählen des bereit-zur-Ablation-Menüs (12) und eines Bestätigungs-Untermenüs (14), Drücken und Loslassen des Fußpedals zum Starten der Ablation der Gewebemasse und Drücken und Loslassen des Fußpedals zum Stoppen der Ablation der Gewebemasse erreicht wird.

12. System nach Anspruch 1, wobei die Ablation der Gewebemasse durch Emission von Radiofrequenzenergie von jedem der Stylets (20) bewirkt wird, die bewirkt, dass die Temperatur in der Gewebemasse, die an die Stylets angrenzt, auf eine voreingestellte Zieltemperatur (26) ansteigt, die von der Vielzahl der Stylets (20) gemessen wird.

13. System nach Anspruch 12, wobei jede der Vielzahl von Stylets (20) auf der graphischen Benutzerschnittstelle (10) die Temperatur der Gewebemasse angrenzend an jede der Vielzahl von Stylets (20) anzeigt, und wobei eine Durchschnittstemperatur (24) der Gewebemasse aus den Temperaturen berechnet wird, die von zwei oder mehr der Vielzahl von Stylets (20) angrenzend an die Gewebemasse angezeigt werden, wobei die Durchschnittstemperatur (24) auf der graphischen Benutzerschnittstelle (10) angezeigt wird.

## Revendications

1. Système de commande à distance d'un dispositif chirurgical d'ablation par radiofréquence, le système comprenant :
(a) un premier dispositif d'imagerie (228) d'un premier type ayant une première sortie image (232),
ledit premier dispositif d'imagerie (228) étant positionné pour représenter en images une zone étant soumise à une chirurgie ;
(b) un deuxième dispositif d'imagerie (338) d'un deuxième type ayant une deuxième sortie image (340), ledit deuxième dispositif d'imagerie (338) étant positionné pour représenter en images ladite zone étant soumise à une chirurgie ;
(c) un ordinateur (112) couplé pour recevoir lesdites première (232) et deuxième (340) sorties
image et combiner lesdites première (232) et deuxième (340) sorties image en une sortie image unitaire représentant une image unitaire ;
(d) un programme logiciel informatique (116), installé dans ledit ordinateur (112) pour générer une interface utilisateur graphique (10) incluant des éléments de menu (12) et sous-menu (14) ;
(e) un dispositif chirurgical d'ablation par radiofréquence (16) couplé audit ordinateur (112), ledit dispositif d'ablation (16) constitué d'une poignée d'opérateur (3) à une extrémité, et un trocart (5) et une pluralité de mandrins (20) à une extrémité opposée ;
(f) une matrice de boutons de navigation constituée d'un système de contrôle électronique à deux boutons monté sur la poignée d'opérateur (3) du dispositif d'ablation (16), ledit système de contrôle électronique à deux boutons est constitué d'un bouton de défilement (23) et d'un bouton de sélection (25) qui interagit avec le logiciel informatique (116) affiché sur l'interface utilisateur graphique (10) ;
(g) le logiciel (116), installé dans ledit ordinateur (112), pour recevoir et afficher
des informations reçues dudit dispositif chirurgical (16) et/ou pour contrôler le fonctionnement dudit dispositif chirurgical (16) ; et
(h) un écran couplé audit ordinateur (112) pour afficher ladite interface utilisateur graphique (10) et ladite image unitaire ;
(i) le trocart (5), la pluralité de mandrins (20) et le système de contrôle électronique à deux boutons avec le bouton de défilement (23) et le bouton de sélection (25), chacun de ladite pluralité de mandrins (20) ayant un volume interne qui contient un transducteur de thermocouple à fil pour la mesure de la température, ladite pluralité de mandrins (20) employant des rayonnements de radiofréquence d'une source à radiofréquences (226), dans lequel un courant électrique se déplace des mandrins (20) du dispositif d'ablation (16) à travers une masse de tissu du patient et vers un coussinet droit et gauche (34) placés, respectivement, au sommet de la jambe droite et gauche du patient, la température plus élevée atteinte par la masse de tissu adjacente aux mandrins (20) du dispositif d'ablation (16) dans chacun des coussinets droit et gauche (34) étant affichée sur l'interface utilisateur graphique (10) comme un élément de température de coussinet droit et gauche, dans lequel la pression du bouton de défilement (23) fait défiler à travers les éléments de menu (12) associés aux éléments de sous-menu (14) affichés sur l'interface utilisateur graphique, et la pression du bouton de sélection (25) sélectionne un élément de menu désiré (12) ou un élément de sous-menu (14) ;
(j) dans lequel le premier dispositif d'imagerie (228) comprend une sonde à ultrasons ayant une sortie image à ultrasons et qui est configurée pour produire une image de la zone étant soumise à une chirurgie de sorte que la sortie image à ultrasons d'une région interne de la zone soit affichée en relation avec une image représentative du dispositif chirurgical d'ablation et une interface utilisateur graphique ;
(k) dans lequel le deuxième dispositif d'imagerie (338) comprend un appareil photo laparoscopique ayant une sortie image laparoscopique qui est configuré pour être positionné afin de produire une image d'une région de ladite zone étant soumise à une chirurgie ; et
(l) dans lequel l'ordinateur (112) est couplé pour recevoir et combiner ladite sortie image à ultrasons et ladite sortie image laparoscopique dans l'image unitaire pour l'affichage sur ledit écran d'interface utilisateur graphique
de sorte que la sortie image à ultrasons et la sortie image laparoscopique soient superposées l'une sur l'autre.

2. Système selon la revendication 1, comprenant en outre la pression d'une pédale de la part du praticien pour envoyer les rayonnements de radiofréquence à travers le trocart (5) et/ou la pluralité de mandrins (20) du dispositif d'ablation (16) afin d'éliminer ou faire coaguler la masse de tissu du patient.

3. Système selon la revendication 2, dans lequel le trocart (5) et la pluralité de mandrins sont déployés de manière adjacente à la masse de tissu dans le patient.

4. Système selon la revendication 1, dans lequel les séries de menus (12) sont sélectionnés dans le groupe consistant en données du fibrome, descripteurs, sommaire, procédure de sélection, options de « prêt à l'ablation » et « prêt à la coag. ».

5. Système selon la revendication 4, dans lequel le menu de données du fibrome inclut des options de sous-menu (14) sélectionnées dans le groupe consistant en nombre (14a), diamètre (14b), position (14c) et type (14d).

6. Système selon la revendication 5, dans lequel les options de position (14c) sont sélectionnées dans le groupe consistant en positions avant, arrière et latérale (14C).

7. Système selon la revendication 6, dans lequel les options de positions avant, arrière et latérale (14C) incluent chacune des sous-menus sélectionnés dans le groupe consistant en options de ligne centrale, droite et gauche (14D).

8. Système selon la revendication 7, dans lequel le sous-menu pour les options de ligne centrale, droite et gauche (14D) inclut pour chacune un sous-menu ultérieur sélectionné dans le groupe consistant en options de fond utérin, à moitié de l'utérus, de segment inférieur et cervicales.

9. Système selon la revendication 8, dans lequel le sous-menu pour les options de fond utérin, à moitié de l'utérus, de segment inférieur et cervicales (14E) inclut pour chacune un sous-menu ultérieur sélectionné dans le groupe consistant en options intramurale, sous-séreuse, sous-muqueuse 1 et sous-muqueuse 2 (14F).

10. Système selon la revendication 4, dans lequel le menu de procédure de sélection (12) inclut les sous-menus (14) sélectionnés dans le groupe consistant en options de température, manuelle et d'impédance.

11. Système selon la revendication 4, dans lequel l'ablation de la masse de tissu au moyen du dispositif d'ablation (16) est achevée en sélectionnant le menu «prêt à l'ablation » (12) et un sous-menu de validation (14), en pressant et relâchant la pédale pour commencer l'ablation de la masse de tissu et en pressant et relâchant la pédale pour fermer l'ablation de la masse de tissu.

12. Système selon la revendication 1, dans lequel l'ablation de la masse de tissu est effectuée par l'émission d'énergie de radiofréquence de chaque mandrin (20), ce qui induit la température dans la masse de tissu adjacente aux mandrins à augmenter jusqu'à une température cible prédéfinie (26) mesurée par la pluralité de mandrins (20).

13. Système selon la revendication 12, dans lequel chacun de la pluralité de mandrins (20) affiche sur l'interface utilisateur graphique (10) la température de la masse de tissu adjacente à chacun de la pluralité de mandrins (20), et dans lequel une température moyenne (24) de la masse de tissu est calculée parmi les températures indiquées par deux ou plusieurs de la pluralité de mandrins (20) adjacents à la masse de tissu, ladite température moyenne (24) affichée sur l'interface utilisateur graphique (10).
